# EUROPEAN PATENT SPECIFICATION

(11) **EP 0 579 280 B1**
(45) Date of publication and mention of the grant of the patent: **28.02.1996**
(21) Application number: 93115360.5
(22) Date of filing: 19.12.1986
(51) Int. Cl.: A01N 43/56, A01N 55/00, C07D 231/38, A61K 31/415, C07F 7/08, C07D 231/44, C07D 231/40, C07D 231/18, C07D 231/16, C07D 231/14, C07D 231/12

(54) **Pesticidal method using N-phenylpyrazoles**
Pestizides-Verfahren unter Verwendung von N-Phenylpyrazolen
Méthode pesticide utilisant des N-phénylpyrazoles

(30) Priority: 20.12.1985 GB 8531485
(43) Date of publication of application: 19.01.1994
(62) Divisional of application: 86309981.8
(73) Proprietor: RHONE-POULENC AGROCHIMIE, F-69009 Lyon (FR)
(72) Inventor: Hatton, Leslie Roy, Dagenham, Essex RM10 7XS (GB); Hawkins, David William, Dagenham, Essex RM10 7XS (GB); Parnell, Edgar William, Dagenham, Essex RM10 7XS (GB); Pearson, Christopher John, Dagenham, Essex RM10 7XS (GB); Roberts, David Alan, Dagenham, Essex RM10 7XS (GB)
(74) Representative: Bentham, Stephen

(56) References cited:
- EP-A- 0 200 872
- EP-A- 0 201 852
- AU-A- 508 225
- US-A- 2 998 419
- US-A- 3 423 424
- US-A- 3 760 084
- US-A- 4 145 554
- JOURNAL OF HETROCYCLIC CHEMISTRY, vol. 12, no. 12, December 1975, pages 1199- 1205, Provo, Utah, US; P.L. SOUTHWICK et al.: "Preparation of 4,6- diaminopyrazolo(3,4-d)pyrimidines with variations in substitution at the 1- and 3-positions(1)"
- IL FARMACO ED. SCIENTIFICA, vol. XXXVIII, no. 4, April 1983, pages 274-282, Pavia, IT; P. GIORI et al.: "Synthesis and antifungal activity of pyrazole derivatives containing sulfurated functions"
- CHEMICAL ABSTRACTS, vol. 91, no. 25, 17th December 1979, page 181, abstract no. 205651p, Columbus, Ohio, US; & JP-A- 79 84 032 (ISHIHARA SANGYO KAISHA LTD)

## Description

This invention relates to the use of N-phenylpyrazole derivatives against arthropod, plant nematode and helminth pests, to compositions containing them and to novel N-phenylpyrazole derivatives.

In J. Heter. Chem., 12 (1975), 1199-1205, P.L. Southwick and B. Dhawan have described experiments for the preparation of 4,6-diamino-pyrazolo[3,4-d]pyrimidines in the expectation that such pyrimidine derivatives would have useful pharmacological properties. They employed as starting materials 5-amino-4-cyanopyrazoles carrying on the 1-position a hydrogen atom, a methyl group, a hydroxyethyl group or a phenyl group substituted by one or more chlorine atoms and/or methyl groups, and on the 3-position a hydrogen atom, a methyl group or a phenyl or benzyl group. This publication contains no suggestion that compounds of general formula I possess or would be expected to possess activity against arthropods, helminths or plant nematodes.

Apparently these pyrazole compounds did not lead (according to the authors of the article) to useful therapeutic (viz. antimalarial) 4,6-diaminopyrazolo[3,4-d]pyrimidines.

US Patent No. 3760084 describes certain 5-amino-1-phenyl-pyrazoles as being useful for ameliorating inflammation in warm-blooded animals: the compounds carry on the 3-position hydrogen or a lower alkyl group and on the 4-position a carbamoyl or cyano group.

US Patent No. 3869274 describes certain 4-nitropyrazoles as being useful for the induction of abscission of fruit from fruit-bearing plants.

US Patent No. 4066776 describes a very extensive group of 1,4-disubstituted-3-nitropyrazoles as having antimicrobial, parasiticidal and herbicidal properties: the great biological activity of the compounds is stated to be limited to the 3-nitropyrazoles disclosed, the characterising feature of the compounds being the 3-nitropyrazole nucleus.

Japanese Patent Publication No. 12644/64 describes a process for the preparation of 4-thiocyanatopyrazole derivatives which are stated to be useful as germicides.

Japanese Patent Publication No. 49-117502 describes certain pyrazole sulphonamides having anti-thrombogenic properties.

None of the foregoing publications describes or suggests that compounds of general formula I possess or would be expected to possess the activity against arthropods, helminths or plant nematodes which has been discovered by the Applicants.

It has now unexpectedly been found after extensive research and experimentation that the N-phenylpyrazole derivatives of the general formula I depicted hereinafter wherein Y represents a halogen, i.e. fluorine, chlorine, bromine or iodine, atom, the cyano group or a group RSO₂, RSO or RS in which R represents a straight- or branched-chain alkyl group containing from 1 to 6 carbon atoms which may be unsubstituted or substituted by one or more halogen atoms, a cycloalkyl group containing from 3 to 5 carbon atoms, a straight- or branched-chain alkenyl group containing from 2 to 6 carbon atoms, the thiocyanato group, the sulphamoyl group which may be unsubstituted or substituted by one or two straight- or branched-chain alkyl groups which may be the same or different and contain from 1 to 6 atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms, a straight- or branched-chain alkanoyl group containing from 2 to 7 carbon atoms, or a straight- or branched-chain alkyl group containing from 1 to 6 carbon atoms which may be unsubstituted or substituted by one or more halogen atoms, Z represents the hydrogen atom, or the amino group -NR¹R² wherein R¹ and R², which may be the same or different, each represents a hydrogen atom or a straight- or branched-chain alkyl group (containing from 1 to 6 carbon atoms, and which may be unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contain from 2 to 7 carbon atoms or together form a 5 or 6 membered cyclic imide with the nitrogen atom to which they are attached and themselves may be unsubstituted or substituted by one or more halogen atoms) or cycloalkylcarbonyl group (which contain from 4 to 7 carbon atoms) or straight-or branched-chain alkoxycarbonyl groups (which contain from 2 to 7 carbon atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which may be unsubstituted or substituted on methylene by a straight- or branched-chain alkyl group containing from 1 to 4 carbon atoms, or represents a halogen, i.e. fluorine, chlorine, bromine or iodine, atom, a straight- or branched-chain alkyl group containing from 1 to 4 carbon atoms, the carboxy group, or a straight- or branched-chain alkylthio, alkylsulphinyl or alkylsulphonyl group containing from 1 to 6 carbon atoms which may be unsubstituted or substituted by one or more halogen atoms, or represents a straight- of branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, R³ represents a halogen, i.e. fluorine, chlorine, bromine or iodine atom, a straight- or branched-chain alkyl or alkoxy group containing from 1 to 4 carbon atoms which may be unsubstituted or substituted by one or more halogen atoms, (e.g. a trifluoromethyl or trifluoromethoxy group), a straight- or branched-chain alkylthio or alkylsulphinyl group containing from 1 to 4 carbon atoms which is substituted by one or more halogen atoms (e.g. a trifluoromethylthio or trifluoromethylsulphinyl group), the nitro or cyano group or a straight- or branched-chain alkylsulphonyl group containing from 1 to 4 carbon atoms which may be unsubstituted or substituted by one or more halogen atoms (e.g. the trifluoromethylsulphonyl group), and R⁴ represents a cycloalkyl group containing from 3 to 6 carbon atoms, and n represents an integer from 1 to 5 inclusive, and, when Z represents a carboxy group, salts thereof with pesticidally-acceptable bases, have valuable activity against arthropod, plant nematode and helminth pests, more particularly by ingestion of the compound(s) of general formula I by the arthropods. When n represents an integer from 2 to 5 inclusive, atoms and groups represented by R³ may be the same or different.

By the term 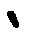 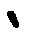 salts with pesticidally acceptable bases' is meant salts the cations of which are known and accepted in the art for the formation of salts of pesticidally active acids for agricultural or horticultural use. When intended for application to vertebrates to combat infection or infestation by arthropods or helminths, the salts with bases used will be non-toxic. By the term 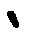 non-toxic' is meant salts with bases the cations of which are innocuous to the vertebrates at the doses administered and which do not vitiate the beneficial effects produced by the anion.

Preferably, the salts are water-soluble. Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts. It is to be understood that where reference is made in the present specification to the compounds of general formula I such reference is intended to include also the salts with pesticidally acceptable bases of compounds of general formula I where appropriate.

Preferred compounds of general formula I are those with phenyl substitution which is 2,4,6-trichloro, 2,3,5,6-tetrachloro, 2-chloro-4-trifluoromethyl, 2,3,5,6-tetrafluoro-4-trifluoromethyl, 2,6-dichloro-4-trifluoromethylthio, 2-chloro-3,5,6-trifluoro-4-trifluoromethyl, 2,6-dichloro-3,5-difluoro-4-trifluoromethyl, 2,6-dichloro-4-nitro, 2,6-dichloro-4-trifluoromethylsulphinyl, 2,6-dichloro-4-methanesulphonyl and 2,6-dichloro-4-trifluoromethanesulphonyl.

Compounds of general formula I wherein (R³)n represents 2,6-dichloro-4-trifluoromethyl or 2,6-dichloro-4-trifluoromethoxy substitution of the phenyl group are especially preferred.

Preferred compounds are those where R⁴ represents a cycloalkyl group, Y represents a halogen atom, the cyano group, a group RSO₂, RSO or RS, the thiocyanato group, a sulphamoyl group, a carbamoyl group, an
alkoxycarbonyl group, an alkanoyl group or an alkyl group which is unsubstituted or substituted by one or more halogen atoms,
Z represents the hydrogen atom, the amino group -NR¹R² or an alkylsulphenylamino group, an alkoxymethyleneamino group which is unsubstituted or substituted on methylene by an alkyl group, a halogen atom, an alkyl group, the carboxy group, an alkylthio, alkylsulphinyl or alkylsulphonyl group which is optionally halogen substituted, a trialkylsilylmethyl group, a trialkylsilyl group or the cyano or nitro group.

It will be appreciated that the groups listed above are as hereinbefore defined earlier in the specification.

Compounds of general formula I which are of particular interest against arthropods are:
1 5-Amino-4-cyano-3-cyclopropyl-1-(2,6-dichloro-4-trifluoromethylphenyl)pyrazole

According to a feature of the present invention, there is provided a method for the control of arthropod, plant nematode or helminth pests at a locus which comprises the treatment of the locus (e.g. by application or administration) with an effective amount of a compound of general formula I, or a pesticidally acceptable salt thereof, wherein the various symbols are as hereinbefore defined, with the exclusion of a method for the treatment of the human or animal body carried out by a medical or veterinary practitioner as therapy. The compounds of general formula I may, in particular, be used in the fields of veterinary medicine and livestock husbandry and in the maintenance of public health against arthropods or helminths which are parasitic internally or externally upon vertebrates, particularly warm-blooded vertebrates, for example man and domestic animals, e.g. cattle, sheep, goats, equines, swine, poultry, dogs, cats and fishes, for example Acarina, including ticks (e.g. Ixodes spp., Boophilus spp. e.g. Boophilus microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata) and mites (e.g. Damalinia spp., Dermahyasus gallinae, Sarcoptes spp. e.g. Sarcoptes scabiei, Psoroptes spp., Chorioptes spp., Demodex spp., Eutrombicula spp.,); Diptera (e.g. Aedes spp., Anophelea spp., Musca spp., Hypoderma spp., Gasterophilus spp., Simulium spp.); Hemiptera (e.g. Triatoma spp.); Phthiraptera (e.g. Damalinia spp., Linognathus spp.); Siphonaptera (e.g. Ctenocephalides spp.); Dictyoptera (e.g. Periplaneta spp., Blatella spp.); Hymenoptera (e.g. Monomorium pharaonis); for example against infections of the gastro-intestinal tract caused by parasitic nematode worms, for example members of the family Trichostrongylidae, Nippostrongylus brasiliensis, Trichinella spiralis, Haemonchus contortus, Trichostrongylus colubriformis, Nematodirus battus, Ostertagia circumcincta, Trichostrongylus axei, Cooperia spp. and Hymenolepis nana; in the protection of stored products, for example cereals, including grain and flour, groundnuts, animal feedstuffs, timber and household goods, e.g. carpets and textiles, against attack by arthropods, more especially beetles, including weevils, moths and mites, for example Ephestia spp. (flour moths), Anthrenus spp. (carpet beetles), Tribolium spp. (flour beetles), Sitophilus spp. (grain weevils) and Acarus spp. (mites), in the control of cockroaches, ants and similar arthropod pests in infested domestic and industrial premises and in the control of mosquito larvae in waterways, wells, reservoirs or other running or standing water; in agriculture, against adults, larvae and eggs of Lepidoptera (butterflies and moths), e.g. Heliothis spp. such as Heliothis virescens (tobacco budworm), Heliothis armigera and Heliothis zea, Spodoptera spp. such as S.exempta, S.littoralis (Egyptian cotton worm), S.eridania (southern army worm), Mamestra configurata (bertha army worm); Earias spp. e.g. E.insulana (Egyptian bollworm), Pectinophora spp. e.g. Pectinophora gossypiella (pink bollworm), Ostrinia spp. such as O.nubilalis (European cornborer), Trichoplusia ni (cabbage looper), Pieris spp. (cabbage worms), Laphygma spp. (army worms), Agrotis and Amathes spp. (cutworms), Wiseana spp. (porina moth), Chilo spp. (rice stem borer), Tryporyza spp. and Diatraea spp. (sugar cane borers and rice borers), Sparganothis pilleriana (grape berry moth), Cydia pomonella (codling moth), Archips spp. (fruit tree tortrix moths), Plutella xylostella (diamond back moth); against adults and larvae of Coleoptera (beetles) e.g. Hypothenemua hampei (coffee berry borer), Hylesinus spp. (bark beetles), Anthonomus grandis (cotton boll weevil), Acalymma spp. (cucumber beetles), Lema spy., Psylliodes spp., Leptinotarsa decemlineata (Colorado potato beetle), Diabrotica spp. (corn rootworms), Gonocephalum spp. (false wire worms), Agriotes spp. (wireworms), Dermolepida and Heteronychus spp. (white grubs), Phaedon cochleariae (mustard beetle), Liasorhoptrus oryzophilus (rice water weevil), Meligethes spp. (pollen beetles), Ceutorhynchus spp., Rhynchophurus and Cosmopolites spp. (root weevils); against Hemiptera e.g. Psylla spp., Bemisia spp., Aphis spp., Myzus spp., Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Psuedococcua spp., Helopeltis spp. (mosquito bugs), Lygus spp., Dysdercus spp., Oxycarenus spp., Nezara spp.; Hymenoptera e.g. Athalia spp. and Cephus spp. (saw flies), Atta spp. (leaf cutting ants); Diptera e.g. Hylemyia spp. (root flies), Atherigona spp. and Chlorops spp. (shoot flies), Phytomyza spp. (leaf miners), Ceratitis spp. (fruit flies); Thysanoptera such as Thrips tabaci; Orthoptera such as Locusta and Schistocerca spp. (locusts) and cricketa e.g. Gryllus spp. and Acheta spp.; Collembola e.g. Sminthurus spp. and Onychiurus spp. (springtails), Isoptera e.g. Odontotermes spp. (termites), Dermaptera e.g. Forficula spp. (earwigs) and also other arthropods of agricultural significance such as Acari (mites) e.g. Tetranychus spp., Panonychus spp. and Bryobia spp. (spider mites), Eriophyes spp. (gall mites), Polyphagotarsonemus spp.; Blaniulus spp. (millipedes), Scutigerella spp. (symphilida), Oniscus spp. (woodlice) and Triops spp. (cruatacea); nematodes which attack plants and trees of importance to agriculture, forestry, horticulture either directly or by spreading bacterial, viral, mycoplasma or , fungal diseases of the plants, root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g. R. reniformis); Rotylenchus spp. (e.g. R. robustus); Helicotylenchus spp (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp. (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

The invention also provides a method for the control of arthropod or nematode pests of plants which comprises the application to the plants or to the medium in which they grow of an effective amount of a compound of general formula I or a pesticidally acceptable salt thereof.

The compounds of general formula I may be applied in solid or liquid compositions to the soil principally to control those nematodes dwelling therein but also to the foliage principally to control those nematodes attacking the aerial parts of the plants (e.g. Aphelenchoides spp. and Ditylenchus spp. listed above).

The compounds of general formula I are of value in controlling pests which feed on parts of the plant remote from the point of application, e.g. leaf feeding insects are killed by the subject compounds applied to roots.

In addition the compounds may reduce attacks on the plant by means of antifeeding or repellant effects.

The compounds of general formula I are of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids).

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compounds of general formula I are of particular value in the control of arthropods or helminths which are injurious to, or spread or act as vectors of diseases in man and domestic animals, for example those hereinbefore mentioned, and more especially in the control of ticks, mites, lice, fleas, midges and biting, nuisance and myiasis flies. The compounds of general formula I are particularly useful in controlling arthropods or helminths which are present inside domestic host animals or which feed in or on the skin or suck the blood of the animal, for which purpose they may be administered orally, parenterally, percutaneously or topically.

The compositions hereinafter described for topical application to man and animals and in the protection of stored products, household goods, property and areas of the general environment may, in general, alternatively be employed for application to growing crops and crop growing loci and as a seed dressing.

Suitable means of applying the compounds of general formula I include:-
to persons or animals infested by or exposed to infestation by arthropods or helminths, by parenteral, oral or topical application of compositions in which the active ingredient exhibits an immediate and/or prolonged action over a period of time against the arthropods or helminths, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax-smears and livestock self-treatment systems;
to the environment in general or to specific locations where pests may lurk, including stored products, timber, household goods, and domestic and industrial premises, as sprays, fogs, dusts, smokes, wax-smears, lacquers, granules and baits, and in tricklefeeds to waterways, wells, reservoirs and other running or standing water; to domestic animals in feed to control fly larvae feeding in their faeces.

The compounds of general formula I may be applied to control arthropods or helminths in compositions of any type known to the art suitable for internal or external administration to vertebrates or application for the control of arthropods in any premises or indoor or outdoor area, containing as active ingredient at least one compound of general formula I in association with one or more compatible diluents or adjuvants appropriate for the intended use. All such compositions may be prepared in any manner known to the art.

Compositions suitable for administration to vertebrates or man include preparations suitable for oral, parenteral, percutaneous, e.g. pour-on, or topical administration.

Compositions for oral administration comprise one or more of the compounds of general formula I in association with pharmaceutically acceptable carriers or coatings and include, for example, tablets, pills, capsules, pastes, gels, drenches, medicated feeds, medicated drinking water, medicated dietary supplements, slow-release boluses or other slow-release devices intended to be retained within the gastro-intestinal tract. Any of these may incorporate active ingredient contained within microcapsules or coated with acid-labile or alkali-labile or other pharmaceutically acceptable enteric coatings. Feed premixes and concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle and solid or semisolid subcutaneous implants or pellets designed to release active ingredient over a protracted period and may be prepared and made sterile in any appropriate manner known to the art.

Compositions for percutaneous and topical administration include sprays, dusts, baths, dips, showers, jets, greases, shampoos, creams, wax-smears, or pour-on preparations and devices (e.g. ear tags) attached externally to animals in such a way as to provide local or systemic arthropod control.

Solid or liquid baits suitable for controlling arthropods comprise one or more compounds of general formula I and a carrier or diluent which may include a food substance or some other substance to induce consumption by the arthropod.

Liquid compositions include water miscible concentrates, emulsifiable concentrates, flowable suspensions, wettable or soluble powders containing one or more compounds of general formula I which may be used to treat substrates or sites infested or liable to infestation by arthropods including premises, outdoor or indoor storage or processing areas, containers or equipment and standing or running water.

Solid homogenous or heterogenous compositions containing one or more compounds of general formula I, for example granules, pellets, briquettes or capsules, may be used to treat standing or running water over a period of time. A similar effect may be achieved using trickle or intermittent feeds of water dispersible concentrates as described herein.

Compositions in the form of aerosols and aqueous or non-aqueous solutions or dispersions suitable for spraying, fogging and low- or ultra-low volume spraying may also be used.

Suitable solid diluents which may be used in the preparation of compositions suitable for applying the compounds of general formula I include aluminium silicate, kieselguhr, corn husks, tricalcium phosphate, powdered cork, adsorbent carbon black, magnesium silicate, a clay such as kaolin, bentonite or attapulgite, and water soluble polymers and such solid compositions may, if desired, contain one or more compatible wetting, dispersing, emulsifying or colouring agents which, when solid, may also serve as diluent.

Such solid compositions, which may take the form of dusts, granules or wettable powders, are generally prepared by impregnating the solid diluents with solutions of the compound of general formula I in volatile solvents, evaporating the solvents and, if necessary, grinding the products so as to obtain powders and, if desired, granulating or compacting the products so as to obtain granules, pellets or briquettes or by encapsulating finely divided active ingredient in natural or synthetic polymers, e.g. gelatin, synthetic resins and polyamides.

The wetting, dispersing add emulsifying agents which may be present, particularly in wettable powders, may be of the ionic or non-ionic types, for example sulphoricinoleates, quaternary ammonium derivatives or products based upon condensates of ethylene oxide with nonyl- and octyl-phenol, or carboxylic acid esters of anhydrosorbitols which have been rendered soluble by etherification of the free hydroxy groups by condensation with ethylene oxide, or mixtures of these types of agents. Wettable powders may be treated with water immediately before use to give suspensions ready for application.

Liquid compositions for the application of the compounds of general formula I may take the form of solutions, suspensions and emulsions of the compounds of general formula I optionally encapsulated in natural or synthetic polymers, and may, if desired, incorporate wetting, dispersing or emulsifying agents. These emulsions, suspensions and solutions may be prepared using aqueous, organic or aqueous-organic diluents, for example acetophenone, isophorone, toluene, xylene, mineral, animal or vegetable oils, and water soluble polymers (and mixtures of these diluents), which may contain wetting, dispersing or emulsifying agents of the ionic or non-ionic types or mixtures thereof, for example those of the types described above. When desired, the emulsions containing the compounds of general formula I may be used in the form of self-emulsifying concentrates containing the active substance dissolved in the emulsifying agents or in solvents containing emulsifying agents compatible with the active substance, the simple addition of water to such concentrates producing compositions ready for use.

Compositions containing compounds of general formula I which may be applied to control arthropod, plant nematode or helminth pests, may also contain synergists (e.g. piperonyl butoxide or sesamex), stabilizing substances, other insecticides, acaricides, plant nematocides or anthelmintics, fungicides (agricultural or veterinary as appropriate e.g. benomyl, iprodione), bactericides, arthropod or vertebrate attractants or repellants or pheromones, reodorants, flavouring agents, dyes and auxiliary therapeutic agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjuntion with, the compositions of the present invention are:-acephate, chlorpyrifos, demeton-S-methyl, disulfoton, ethoprofos, fenitrothion, malathion, monocrotophos, parathion, phosalone, pirimiphos-methyl, triazophos, cyfluthrin, cypermethrin, deltamethrin, fenpropathrin, fenvalerate, permethrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectin, milbemycins, thiophanate, trichlorfon and dichlorvos.

The compositions for application to control arthropods usually contain from 0.00001% to 95%, more particularly from 0.0005% to 50%, by weight of one or more compounds of general formula I or of total active ingredients (that is to say the compound(s) of general formula I together with other substances toxic to arthropods and plant nematodes, anthelmintics, synergists, trace elements or stabilisers). The actual compositions employed and their rate of application will be selected to achieve the desired effects(s) by the farmer, livestock producer, medical or veterinary practitioner, pest control operator or other person skilled in the art. Solid and liquid compositions for application topically to animals, timber, stored products or household goods usually contain from 0.00005% to 90%, more particularly from 0.001% to 10%, by weight of one or more compounds of general formula I. For administration to animals orally or parenterally, including percutaneously, solid and liquid compositions normally contain from 0.1% to 90% by weight of one or more compounds of general formula I. Medicated feedstuffs normally contain from 0.001% to 3% by weight of one or more compounds of general formula I. Concentrates and supplements for mixing with feedstuffs normally contain from 5% to 90%, and preferably from 5% to 50%, by weight of one or more compounds of general formula I. Mineral salt licks normally contain from 0.1% to 10% by weight of one or more compounds of general formula I.

Dusts and liquid compositions for application to livestock, persons, goods, premises or outdoor areas may contain 0.0001% to 15%, and more especially 0.005% to 2.0%, by weight of one or more compounds of general formula I. Suitable concentrations in treated waters are between 0.0001 ppm and 20 ppm, and more especially 0.001 ppm to 5.0 ppm, of one or more compounds of general formula I and may also be used therapeutically in fish farming with appropriate exposure times. Edible baits may contain from 0.01% to 5% and preferably 0.01% to 1.0%, by weight of one or more compounds of general formula I.

When administered to vertebrates parenterally, orally or by percutaneous or other means, the dosage of compounds of general formula I wild depend upon the species, age and health of the vertebrate and upon the nature and degree of its actual or potential infestation by arthropods. A single dose of 0.1 to 100 mg, preferably 2.0 to 20.0 mg, per kg body weight of the animal or doses of 0.01 to 20.0 mg, preferably 0.1 to 5.0 mg, per kg body weight of the animal per day for sustained medication are generally suitable by oral or parenteral administration. By use of sustained release formulations or devices, the daily doses required over a period of months may be combined and administered to animals on a single occasion.

The present invention accordingly provides an arthropodical, plant nematocidal or anthelmintic composition which comprises at least one compound of general formula I, or a salt thereof, in association with one or more compatible diluents or carriers.

Medicated feeds which comprise known compounds of general formula I and arthropodicidally- or anthelmintically-acceptable salts thereof and an edible carrier or diluent form a feature of the present invention.

The following Examples illustrate compositions for use against arthropod, plant nematode or helminth pests which comprise, as active ingredient, compounds of general formula I.

### Example A

A dusting powder may be prepared by intimately mixing:-

| | |
|---|---|
| Compound of general formula I | 1 to 10% w/w (weight/weight) |
| Talc superfine | to 100% by weight |

This powder may be applied to a locus of arthropod infestation, for example refuse tips or dumps, stored products or household goods or animals infested by, or at risk of infestation by, arthropods to control the arthropods by oral ingestion. Suitable means for distributing the dusting powder to the locus of arthropod infestation include mechanical blowers, handshakers and livestock self treatment devices.

### Example B

An edible bait may be prepared by intimately mixing:-

| | |
|---|---|
| Compound of general formula I | 0.1 to 1.0% w/w |
| Wheat flour | 80% w/w |
| Molasses | to 100% w/w |

This edible bait may be distributed at a locus, for example domestic and industrial premises, e.g. kitchens, hospitals or stores, or outdoor areas, infested by arthropods, for example ants, locusts, cockroaches and flies, to control the arthropods by oral ingestion.

### Example C

A solution may be prepared containing:-

| | |
|---|---|
| Compound of general formula I | 15% w/v (weight/volume) |
| Dimethylsulphoxide | to 100% by volume |

by dissolving the pyrazole derivative in a portion of the dimethylsulphoxide and then adding more dimethylsulphoxide to the desired volume. This solution may be applied to domestic animals infested by arthropods, percutaneously as a pour-on application or, after sterilisation by filtration through a polytetrafluoroethylene membrane (0.22 µm pore size), by parenteral injection, at a rate of application of from 1.2 to 12 ml of solution per 100 kg of animal body weight.

### Example D

A wettable powder may be formed from:-

| | |
|---|---|
| Compound of general formula I | 50% w/w |
| Ethylan BCP (a nonylphenol/ethylene oxide condensate containing 9 moles of ethylene oxide per mol of phenol) | 5% w/w |
| Aerosil (silicon dioxide of microfine-particle size) | 5% w/w |
| Celite PF (synthetic magnesium silicate carrier) | 40% w/w |

by adsorbing the Ethylan BCP onto the Aerosil, mixing with the other ingredients and grinding the mixture in a hammer-mill to give a wettable powder, which may be diluted with water to a concentration of from 0.001% to 2% w/v of the pyrazole compound and applied to a locus of infestation by arthropods, for example dipterous larvae, or plant nematodes by spraying, or to domestic animals infested by, or at risk of infestation by, arthropods, by spraying or dipping, or by oral administration as drinking water, to control the arthropods or helminths.

### Example E

A slow release bolus may be formed from granules containing a density agent, binder, slow-release agent and a compound of general formula I at varying percentage compositions. By compressing the mixture a bolus with a specific gravity of 2 or more can be formed and may be administered orally to ruminant domestic animals for retention within the reticulo-rumen to give a continual slow release of pyrazole compound over an extended period of time to control infestation of the ruminant domestic animals by arthropods or helminths.

### Example F

A slow release composition may be prepared from:-

| | |
|---|---|
| Compound of general formula I | 0.5 to 25% w/w |
| polyvinylchloride base | to 100% w/w |

by blending the polyvinylchloride base with the pyrazole compound and a suitable plasticiser, e.g. dioctyl phthalate, and melt-extruding or hot-moulding the homogenous composition into suitable shapes, e.g. granules, pellets, brickettes or strips, suitable, for example, for addition to standing water or, in the case of strips, fabrication into collars or ear-tags for attachment to domestic animals, to control insect pests by slow release of the pyrazole compound.

The compounds of general formula I can be prepared by the application or adaptation of known methods (i.e. methods heretofore used or described in the chemical literature), generally heterocycle formation followed where necessary by changing substituents with protection/deprotection of other substituents if necessary, for example as hereinafter described.

In the following description when symbols appearing in formulae are not specifically defined it is to be understood that they are "as hereinbefore defined" in accordance with the first definition of each symbol in this specification.

Compounds of general formula I conforming to general formula IA wherein Y' represents the cyano group or a group RSO₂, RSO or RS, a straight- or branched-chain alkoxycarbonyl group containing from 2 to 7 carbon atoms, or a straight- or branched-chain alkyl group containing from 1 to 6 carbon atoms which may be unsubstituted or substituted by one or more halogen atoms, Z' represents the unsubstituted amino group or a straight- or branched-chain alkyl group containing from 1 to 4 carbon atoms, and R⁵ represents a cycloalkyl group containing from 3 to 6 carbon atoms, may be prepared by the process which comprises:
(i) the reaction of a compound of the general formula II, or an acid addition salt thereof, e.g. the hydrochloride, with a compound of the general formula III, wherein R⁶ represents the cyano group or a straight- or branched-chain alkanoyl group containing from 2 to 5 carbon atoms and R⁸ represents a straight- or branched-chain alkoxy group containing from 1 to 4 carbon atoms, preferably ethoxy, the hydroxy group or a fluorine, chlorine or bromine atom.
   The reaction of a compound of general formula II with a compound of general formula III (optionally prepared in situ) may be effected in the presence of an inert organic solvent, for example an alkanol containing from 1 to 4 carbon atoms, e.g. ethanol, acetic acid, ethoxyethanol or an ether, and at a temperature from ambient temperature to the reflux temperature of the reaction mixture and optionally in the presence of an alkali metal, e.g. sodium or potassium, acetate, carbonate or bicarbonate or organic base e.g. triethylamine. When an acid addition salt of the compound of general formula II is used, the reaction with the compound of general formula III is effected in the presence of an alkali metal, e.g. sodium or potassium, acetate, carbonate or bicarbonate.
(ii) Compounds of general formula IA wherein Z' represents the unsubstituted amino group may alternatively be prepared directly by reacting a compound of general formula Y'CH₂CN with a compound of general formula II in the presence of a compound of general formula R⁷C(R^{o})₃ wherein R⁷ represents a cycloalkyl group containing from 3 to 6 carbon atoms and R^{o} represents an alkoxy group which may be straight- or branched-chain and preferably contains from 1 to 4 carbon atoms, in an inert organic solvent, preferably ethanol, at a temperature from ambient to reflux.

The compounds of general formula IA may be prepared by reaction of a compound of general formula II with a compound of general formula III with isolation of an intermediate compound of general formula V from the reaction mixture. When the reaction of a compound of general formula II with a compound of general formula III is effected in acetic acid, in the absence or presence of an alkali metal, e.g. sodium or potassium, acetate, the intermediate compound of general formula V may separate from the reaction mixture, depending upon its solubility in the reaction medium, and may, if desired, be isolated before being cyclised as hereinbefore described to a compound of general formula IA. The cyclisation of a compound of general formula V, which constitutes a feature of the invention may be effected in the presence of an inert organic solvent, for example an alkanol containing from 1 to 4 carbon atoms, e.g. ethanol, acetic acid or ethoxyethanol, at a temperature of from ambient temperature up to the reflux temperature of the reaction mixture, and optionally in the presence of sodium ethoxide when the solvent is ethanol.

It will be appreciated that in the preparation of compounds of general formula I the following subsidiary processes or adaptations thereof may be performed in an appropriate combination to achieve the compound sought.

Compounds of general formula I which conform to general formula IB wherein R¹ represents an R⁹C(=O)- group, wherein R⁹ represents a straight- or branched-chain alkyl or alkoxy group containing from 1 to 6 carbon atoms, or a cycloalkyl group containing from 3 to 6 carbon atoms and R² represents a hydrogen atom or an R⁹C(=O)- group which is identical to the group R⁹C(=O)- represented by R¹ or -NR¹R² represents a cyclic imide as hereinbefore defined, may be prepared by the reaction of a compound of general formula I wherein Z represents the unsubstituted amino group, or an alkali metal salt thereof, with a compound of the general formula:-

R⁹COX VI

wherein X represents a chlorine or bromine atom, or with a compound of the general formula:-

(R⁹CO)₂O VII

or with a dicarboxylic acid derivative. The reaction may be conducted in the absence or presence of an inert organic solvent, for example acetonitrile, tetrahydrofuran, a ketone, e.g. acetone, an aromatic hydrocarbon, e.g. benzene or toluene, chloroform, dichloromethane or dimethylformamide, and optionally in the presence of an acid-binding agent, for example pyridine, triethylamine or an alkali metal, e.g. sodium or potassium, carbonate or bicarbonate, at a temperature from 0°C to the reflux temperature of the reaction medium, to give a compound of general formula IB wherein R¹ represents an R⁹C(=O)- group wherein R⁹ is as hereinbefore defined and R² represents a hydrogen atom or an R⁹C(=O)- group, depending upon the reaction conditions chosen and/or the use of an excess of the compound of general formula VI or VII.

Compounds of general formula IB wherein R¹ represents a formyl group and R² represents a hydrogen atom may be prepared by reaction of a compound of general formula I, wherein Z represents the unsubstituted amino group with formic acid. The reaction may be conducted in an inert organic solvent, for example a ketone e.g. methylisobutyl ketone, or an aromatic hydrocarbon, e.g. benzene or toluene, at the reflux temperature of the reaction mixture.

Compounds of general formula IB wherein R¹ represents a formyl group and R² represents a hydrogen atom or a formyl group, may be prepared by the reaction of a compound of general formula I, wherein Z represents the unsubstituted amino group with formylacetic anhydride. Formylacetic anhydride may be prepared from formic acid and acetic anhydride and the reaction with the compound of general formula I may be conducted in the absence or presence of an inert organic solvent, for example a ketone, e.g. acetone, or an aromatic hydrocarbon, e.g. benzene or toluene, and optionally in the presence of an acid-binding agent, for example pyridine, triethylamine or an alkali metal, e.g. sodium or potassium, carbonate or bicarbonate, at a temperature from 0°C to the reflux temperature of the reaction mixture, to give a compound of general formula IB wherein R¹ represents a formyl group and R² represents a hydrogen atom or a formyl group, depending upon the reaction conditions chosen and/or the use of an excess of formylacetic anhydride.

Compounds of general formula IB wherein R¹ represents a formyl group or a group R⁹C(=O)- and R² represents a hydrogen atom may be prepared by the selective removal by hydrolysis of an R⁹C(=O)- group or a formyl group from a compound of general formula IB wherein R¹ and R² both represent a R⁹C(=O) group or a formyl group. Hydrolysis is effected under mild conditions, for example by treatment with an aqueous-ethanolic solution or suspension of an alkali metal, e.g. sodium or potassium, bicarbonate, or with aqueous ammonia.

Compounds of general formula IB wherein R¹ represents a straight- or branched-chain alkoxycarbonyl group containing from 2 to 7 carbon atoms which is unsubstituted or substituted by one or more halogen atoms, and R² represents a hydrogen atom may be prepared by the reaction of a compound of the general formula VIII, wherein R¹⁰ represents an alkoxycarbonyl group R¹¹C(=O), wherein R¹¹ represents a straight- or branched-chain alkoxy group containing from 1 to 6 carbon atoms (which is unsubstituted or substituted by one or more halogen atoms) or a phenoxy group, with a compound of the general formula:-

R¹¹H IX

to replace a first group represented by the symbol R¹⁰ by a hydrogen atom, and to replace the second group represented by the symbol R¹⁰ by an alkoxycarbonyl group when R¹⁰ represents a phenoxycarbonyl group, or, if desired, to replace the second group represented by the symbol R¹⁰ by another alkoxycarbonyl group when R¹⁰ in formula VIII represents an alkoxycarbonyl group. As will be apparent to those skilled in the art, the desired compound of general formula IB is obtained by selection of the appropriate compounds of general formulae VIII and IX. The reaction may be effected in water or an inert aqueous-organic or organic solvent, for example an alkanol containing 1 to 4 carbon atoms, e.g. ethanol, or an aromatic hydrocarbon, e.g. benzene or toluene, or which is preferably an excess of the compound of general formula IX, at a temperature from ambient temperature to the reflux temperature of the reaction mixture and, if necessary, at elevated pressure, and optionally in the presence of a base, for example an alkali metal alkoxide, e.g. of the compound of general formula IX.

Compounds of general formula IB wherein R¹ and R², which may be the same or different, each represents a formyl group or a R⁹C(=O)- group, may be prepared by the reaction of an alkali metal, e.g. sodium or potassium, derivative of a compound of general formula IB wherein R¹ represents a group R⁹C(=O)- as hereinbefore defined, or a formyl group, and R² represents a hydrogen atom with formic acid, formylacetic anhydride or a compound of general formula VI. Reaction may be effected in an inert aprotic solvent, e.g. dimethylformamide, at a temperature from laboratory temperature to the reflux temperature of the reaction mixture.

Alkali metal derivatives of compounds of general formula I (wherein Z represents the unsubstituted amino group) or IB wherein R¹ represents a group R⁹C(=O)- and R² represents a hydrogen atom may be prepared in situ by reaction with an alkali metal, e.g. sodium or potassium, hydride, in an inert aprotic solvent, e.g. dimethylformamide, at a temperature from laboratory temperature to the reflux temperature of the reaction mixture.

Compounds of general formula VIII wherein R¹⁰ represents a group R¹¹C(=O)-, may be prepared as hereinbefore described. Compounds of general formula VIII wherein R¹⁰ represents a phenoxycarbonyl group may be prepared by the reaction of a compound of general formula I (wherein Z represents the unsubstituted amino group), with a compound of the general formula:-

R¹²COX VIA

, wherein R¹² represents a phenoxy group, or with a compound of the general formula:-

(R¹²CO)₂O VIIA

using the reaction conditions hereinbefore described for the reaction of a compound of general formula I with a compound of formula VI or VII.

Compounds of general formula IB wherein R¹ represents a group R¹³ which represents a straight-or branched-chain alkyl group containing from 1 to 6 carbon atoms (which may be unsubstituted or substituted by alkoxycarbonyl groups containing from 2 to 5 carbon atoms) or a cycloalkyl group containing from 3 to 6 carbon atoms, and R² represents a hydrogen atom may be prepared by the removal of the group R⁹C(=O)- of a compound of the general formula IB, wherein R¹ represents a group R¹³ and R² represents a group R⁹C(=O)- . Removal of the group R⁹C(=O)- may be effected by selective hydrolysis under mild conditions, for example by treatment with an alkali metal, e.g. sodium or potassium, hydroxide in water or an inert organic or aqueous-organic solvent, for example a lower alkanol, e.g. methanol, or a mixture of water and lower alkanol, at a temperature from laboratory temperature up to the reflux temperature of the reaction mixture.

Compounds of general formula IB, wherein R¹ represents a group R¹³ and R² represents a group R⁹C(=O)-, may be prepared by reaction of a compound of general formula IB wherein R¹ represents a hydrogen atom , or an alkali metal, e.g., sodium or potassium, derivative thereof, with a compound of the general formula:-

R¹³X¹ X

, wherein X¹ represents a chlorine, bromine or iodine atom. Reaction may be effected in an inert organic solvent, e.g. dichloromethane, tetrahydrofuran, or dimethylformamide, at a temperature from laboratory temperature up to the reflux temperature of the reaction mixture and, when a compound of general formula IB is used, in the presence of a base, e.g. Triton B; or by reaction of a compound of general formula IB wherein R¹ represents the hydrogen atom and R² represents a group R¹³ with a compound of general formula VI or VII.

Compounds of general formula I wherein Z represents an N-(alkyl or cycloalkyl)-N-formylamino group as hereinbefore described may be prepared in a similar manner to the process above using, where appropriate, formylacetic anhydride instead of a compound of general formula VI or VII.

Compounds of general formula IB wherein one of R¹ and R² or both of R¹ and R² represent a straight- or branched- chain alkyl group containing from 1 to 6 carbon atoms or cycloalkyl group containing from 3 to 6 carbon atoms, groups represented by R¹ and R² being identical, may be prepared by reaction of a compound of general formula I, wherein Z represents the unsubstituted amino group, or an alkali metal, e.g. sodium or potassium, derivative thereof, with a compound of general formula X, in the absence or presence of an inert organic solvent, for example an aromatic hydrocarbon, e.g. benzene or toluene, chloroform, dichloromethane, tetrahydrofuran or dimethylformamide, and optionally in the presence of an acid-binding agent, for example pyridine, triethylamine or an alkali metal, e.g. sodium or potassium, bicarbonate, at a temperature from 0°C up to the reflux temperature of the reaction mixture.

Alkali metal derivatives of compounds of formulae IB (wherein R¹ represents a hydrogen atom) and I (wherein Z represents the unsubstituted amino group) may be prepared in situ by the reaction of the compounds, with an alkali metal, e.g. sodium or potassium, hydride, at a temperature from laboratory temperature to the reflux temperature of the reaction mixture.

Compounds of general formula I wherein Z represents a straight- or branched-chain alkoxymethyleneamino group containing from 2 to 5 carbon atoms which may be unsubstituted or substituted on methylene by a straight- or branched-chain alkyl group containing from 1 to 4 carbon atoms may be prepared by the reaction of a compound of general formula I (wherein Z represents the unsubstituted amino group) with a trisalkoxyalkane in the presence of an acidic catalyst, e.g. p-toluenesulphonic acid, at a temperature from ambient temperature to the reflux temperature of the reaction mixture.

Compounds of general formula I, wherein Z represents a straight- or branched-chain, alkylsulphenylamino group containing from 1 to 4 carbon atoms, may be prepared by the reaction of compounds of general formula I (wherein Z represents the unsubstituted amino group) with an alkanesulphenyl chloride in the presence of a base, e.g. sodium hydride, and optionally in the presence of a crown ether catalyst, e.g. 15-crown-5.

The reaction may be performed in a solvent, e.g. tetrahydrofuran, at a temperature from 0°C to the reflux temperature of the reaction mixture.

Compounds of general formula I wherein Z represents -NHCH₂R¹⁴ wherein R¹⁴ represents the hydrogen atom or a straight- or branched-chain alkyl group containing from 1 to 4 carbon atoms may be prepared by reaction of a compound of general formula I wherein Z represents -N=C(OR¹⁵)R¹⁴ wherein R¹⁵ represents a straight- or branched-chain alkyl group containing from 1 to 4 carbon atoms with a reducing agent, preferably sodium borohydride. The reaction may be effected in an inert organic solvent, ethanol or methanol being preferred, at a temperature from 0°C to the reflux temperature of the reaction mixture.

Compounds of general formula I wherein Y represents -C(=O)NH₂ may be prepared by partial hydrolysis of a compound of general formula I wherein Y represents -CN preferably with sulphuric acid at a temperature from ambient temperature to 100°C.

Compounds of general formula I wherein Y represents the chlorine, bromine or iodine atom may be prepared by reaction of a compound of general formula XI with a halogenating agent, preferably N-halosuccinimide in an inert solvent, preferably carbon tetrachloride, at a temperature from ambient temperature to the reflux temperature of the reaction mixture.

Compounds of general formula I wherein Z represents the chlorine, bromine or iodine atom may be prepared by diazotisation of a compound of general formula I wherein Z represents -NH₂ with an alkyl nitrite, preferably tert-butyl nitrite, in the presence of a halogenating agent preferably bromoform, iodine or anhydrous copper chloride at a temperature from 0°C to 100°C.

Compounds of general formula I wherein Y is replaced by the nitro group may be prepared by reacting a compound of general formula XI with a nitrating agent, preferably nitric acid optionally in the presence of sulphuric acid or nitric acid in a solvent such as acetic acid or acetic anhydride at a temperature from 0 C to 100 C.

Compounds of general formula I wherein Y represents -SO₂NR¹⁶R¹⁷ wherein R¹⁶ and R¹⁷, which may be the same or different, each represent the hydrogen atom or a straight- or branched-chain alkyl group containing from 1 to 6 carbon atoms may be prepared by reacting a compound of general formula XIV with an amine of the general formula R¹⁶R¹⁷NH in a solvent such as toluene or water at a temperature from 0°C to the reflux temperature of the reaction mixture.

Compounds of general formula I wherein Y represents -CONR¹⁶R¹⁷ may be prepared by reacting a compound of general formula XV wherein X² represents a chlorine or bromine atom or activated ester moiety e.g. 4-nitrophenoxy group, especially the chlorine atom, with an amine of the general formula R¹⁶R¹⁷NH, in a solvent such as toluene or water, at a temperature from 0°C to the reflux temperature of the reaction mixture.

Intermediates of general formula XI may be prepared by decarboxylation of a compound of general formula XVI, performed by heating at a temperature from 100°C to 250°C optionally in the presence of an inert organic solvent, particularly N,N-dimethylaniline.

Alternatively intermediates of general formula XI may be prepared directly from esters of compounds of general formula XVI by heating in an inert organic solvent preferably acetic acid at a temperature from 50°C to reflux, in the presence of a strong acid preferably hydrobromic acid.

Intermediates of general formula XVI may be prepared by hydrolysis of esters of general formula I wherein Y represents -COOR¹⁸ wherein R¹⁸ represents a straight- or branched- chain alkyl group containing from 1 to 6 carbon atoms, preferably with an alkali metal hydroxide in a solvent such as an aqueous alcohol at a temperature from 0°C to the reflux temperature of the reaction mixture.

Intermediates of general formula XIV may be prepared by reacting a compound of general formula XI with chlorosulphonic acid at a temperature from 0°C to 150°C.

Intermediates of general formula XV are prepared by reacting a compound of general formula XVI with a chlorinating or brominating agent or e.g. 4-nitrophenol (preferably thionyl chloride) at a temperature from ambient temperature to the reflux temperature of the reaction mixture.

Compounds of general formula I wherein Y represents -C(=O)R¹⁸ wherein R¹⁸ represents a straight- or branched-chain alkyl group containing from 1 to 6 carbon atoms may be prepared by the reaction of a compound of general formula XI with an acylating agent such as R¹⁸COCl in the presence of a catalyst such as aluminium chloride and in an inert organic solvent such as 1,1,2,2-tetrachloroethane and at a temperature from 0°C to the reflux temperature of the reaction mixture.

When Z is an amino group it may also be acylated and subsequent hydrolysis using an acid such as hydrochloric or hydrobromic acid in a solvent such as dioxan or acetic acid may be necessary.

Compounds of general formula I wherein Y represents -C(=O)R¹⁸ may also be prepared by the reaction of nitriles of the general formula I wherein Y represents -CN with an organometallic reagent such as a compound of general formula R¹⁸MgX¹ in an inert organic solvent such as diethyl ether or tetrahydrofuran, at a temperature from 0°C to reflux.

Compounds of general formula IC may be prepared by the reaction of a compound of general formula I wherein Y represents the thiocyanato group with an organometallic reagent such as a compound of general formula RMgX¹ in an inert organic solvent such as diethyl ether or tetrahydrofuran, and at a temperature from ambient temperature to the reflux temperature of the reaction mixture.

Compounds of general formula IC wherein RS is other than a 1-alkenylthio group may also be prepared by reacting a compound of general formula I wherein Y represents the thiocyanato group with a base, preferably sodium hydroxide, or a reducing agent, preferably sodium borohydride, in the presence of a reagent of general formula R'X¹ wherein R' is as hereinbefore defined for R with the exclusion of 1-alkenyl groups, for example methyl iodide in an inert organic or aqueous-organic solvent such as an alcohol e.g. ethanol or a mixture of an alcohol and water, the reaction being performed at a temperature from ambient to reflux.

Alternatively compounds of general formula IC wherein RS is other than a 1-alkenylthio group may be prepared by reductive alkylation of disulphides of general formula XVII employing a reducing agent preferably sodium dithionite or sodium borohydride, in the presence of a base, preferably sodium hydroxide or sodium carbonate, and of a reagent of general formula R'X¹ such as methyl iodide in an inert organic or aqueous-organic solvent such as an alcohol e.g. ethanol or a mixture of an alcohol and water, at a temperature from ambient to reflux.

Alternatively compounds of general formula IC may be prepared from a halide of general formula I wherein Y represents a bromine or iodine atom by metal exchange using a strong base, preferably butyl lithium, and subsequent addition of the appropriate disulphide of general formula R-S-S-R in an inert organic solvent such as tetrahydrofuran, and the reaction is performed at a temperature from -78°C to ambient.

Alternatively, compounds of general formula IC wherein RS represents a straight- or branched-chain alkylthio group containing from 1 to 6 carbon atoms which may be unsubstituted or substituted by one or more halogen atoms may be prepared by reacting a compound of general formula XI with an alkanesulphenyl halide (which may be optionally substituted by one or more halogen atoms) in an inert organic solvent, preferably chloroform, in the presence of a base such as pyridine, and at temperatures from 0° to reflux.

Compounds of general formula IC wherein RS represents a methylthio group which is substituted by three halogen atoms which may be the same or different may also be prepared by the reaction of a compound of general formula I wherein Y represents the thiocyanato group with a source of halogenocarbene, such as chloroform and sodium hydroxide, preferably with phase transfer catalysis using for example benzyltriethylammonium chloride or tetrabutylammonium chloride.

Compounds of general formula IC wherein RS represents a straight- or branched-chain alkylthio group containing from 1 to 6 carbon atoms which is substituted by one or more fluorine atoms may also be prepared by a halogen exchange reaction of a compound of general formula IC wherein RS represents a straight- or branched-chain alkylthio group containing from 1 to 6 carbon atoms which is substituted by one or more chlorine atoms with a fluorinating agent such as a mixture of antimony trifluoride and antimony pentachloride, KF or CsF in an aprotic solvent such as sulfolane at a temperature from 50°C to reflux.

Compounds of general formula I wherein Y represents the thiocyanato group may be prepared by the reaction of a compound of general formula XI with a thiocyanating agent such as alkali metal or ammonium salts of thiocyanic acid (e.g. NaSCN) and bromine in an inert organic solvent such as methanol, and at a temperature from 0°C to 100°C.

Intermediates of general formula XVII may be prepared by hydrolysis of thiocyanates of general formula I wherein Y represents the thiocyanato group, preferably using hydrochloric acid in the presence of ethanol at a temperature from ambient to reflux temperature; they may also be prepared by reduction of the thiocyanates by sodium borohydride in an alcohol preferably ethanol at a temperature from ambient to reflux.

Compounds of general formula I wherein Y represents a group RSO may be prepared by the oxidation of compounds of general formula IC by an oxidising reagent preferably 3-chloroperbenzoic acid in an inert organic solvent such as dichloromethane or by hydrogen peroxide in acetic acid at a temperature from 0°C to the reflux temperature of the reaction medium.

Compounds of general formula I wherein Y represents a group RSO₂ may also be prepared by the above process, by employing an excess of the oxidising agent.

Compounds of general formula I wherein Y represents a group RSO₂ wherein R represents a straight- or branched-chain alkyl group containing from 1 to 6 carbon atoms which is substituted by one or more fluorine atoms may also be prepared by a halogen exchange reaction of a compound of general formula I wherein Y represents a group RSO₂ wherein R represents a straight- or branched-chain alkyl group containing from 1 to 6 carbon atoms which is substituted by one or more chlorine atoms with a fluorinating agent such as a mixture of antimony trifluoride and antimony pentachloride, KF or CsF at a temperature from 50°C to 200°C.

Compounds of general formula I wherein Y represents a group RSO₂ may also be prepared by reaction of a compound of general formula XI with the appropriate sulphonic anhydride of general formula (RSO₂)₂O for example trifluoromethanesulphonic or methanesulphonic anhydride and in the presence of aluminium chloride as catalyst, and employing an inert organic solvent such as 1,1,2,2-tetrachloroethane at a temperature from ambient to 150°C.

Compounds of general formula I wherein Z represents a straight- or branched-chain alkyl group containing from 1 to 4 carbon atoms, the carboxy group, a group R¹⁹S wherein R¹⁹ represents a straight- or branched-chain alkyl group containing from 1 to 6 carbon atoms which may be unsubstituted or substituted by one or more halogen atoms or Z represents a trialkylsilyl group containing from 1 to 6 carbon atoms in each alkyl group which may be the same or different may be prepared by the reaction of a compound of general formula I wherein Z represents a hydrogen, bromine or iodine atom with a lithiating agent preferably lithium diisopropylamide or n-butyl lithium, and reaction with the appropriate substrate from alkyl halide, carbon dioxide, dialkylsulphides or trialkylsilyl halides respectively at a temperature from -78°C to ambient temperature, and in an inert solvent, preferably tetrahydrofuran.

Compounds of general formula I wherein Z represents a hydrogen atom may be prepared by diazotisation of an amine of general formula I wherein Z represents the unsubstituted amino group using an alkyl nitrite, preferably tert-butyl nitrite, in an inert solvent preferably tetrahydrofuran, at a temperature from ambient temperature to the reflux temperature of the reaction mixture.

Compounds of general formula I wherein Z represents a group R¹⁹SO may be prepared by the reaction of a compound of general formula I wherein Z represents a group R¹⁹S with an oxidising agent, preferably 3-chloroperbenzoic acid in a solvent such as dichloromethane, or by hydrogen peroxide in acetic acid at a temperature from 0°C to the reflux temperature of the reaction mixture.

Compounds of general formula I wherein Z represents a group R¹⁹SO₂ may also be prepared by the above process, by employing an excess of the oxidising agent.

Compounds of general formula I wherein Z represents the fluorine atom or the cyano group may be prepared by the reaction of a halide of general formula I wherein Z represents the chlorine or bromine atom with an alkali metal fluoride, preferably caesium fluoride, or with an alkali metal cyanide preferably KCN, under anhydrous conditions in an inert solvent, preferably sulfolane, and at a temperature from ambient temperature to 150°C.

Compounds of general formula I wherein Z represents the nitro group may be prepared by oxidation of amines of general formula I wherein Z represents the unsubstituted amino group with an oxidant, preferably trifluoroperacetic acid or m-chloroperbenzoic acid and in an inert organic solvent preferably dichloromethane at a temperature from 0°C to reflux.

Compounds of general formula I wherein Z represents the cyano group may be prepared by dehydration of the corresponding amide preferably by heating with phosphorous pentoxide at a temperature from 50°C to 250°C.

The amides may be prepared (i) by reacting a carboxylic acid of general formula I wherein Z represents a carboxy group with a chlorinating agent preferably thionyl chloride, and (ii) reacting the resultant acid chloride of general formula XVIII with ammonia:-
(i) the reaction with a chlorinating agent preferably thionyl chloride is generally conducted at a temperature from ambient temperature to the reflux temperature of the reaction mixture;
(ii) the reaction with ammonia is generally conducted in a solvent which may be inert, preferably toluene, or in the presence of water, and at a temperature from 0°C to 100°C.

Compounds of general formula I wherein Y represents a group RSO₂ is other than a 1-alkenylsulphonyl group may be prepared alternatively by reaction of sulphinate metal (e.g. sodium) salts with a reagent of general formula R'X¹ or preferably a sulphate of general formula (R')₂SO₄, in a solvent such as water and in the presence of sodium bicarbonate at a temperature from 0°C to 100°C.

The intermediate sulphinate sodium salts may be prepared by reaction of sulphonyl chlorides of general formula XIV with sodium sulphite in the presence of sodium bicarbonate and water as solvent, at a temperature from 50°C to reflux.

Intermediates of general formula XIV may also be prepared from the thiocyanates of general formula I wherein Y represents a thiocyanato group by chlorination using chlorine in a solvent, preferably water, at a temperature from ambient to reflux.

Compounds of general formula I wherein R³ represents a haloalkylsulphinyl group may be prepared by oxidation of a haloalkylthio derivative of general formula I, preferably with m-chloroperbenzoic and in an inert organic solvent preferably dichloromethane, at a temperature from 0°C to reflux.

Compounds of general formula I wherein R³ represents a haloalkylsulphonyl group may be prepared in a similar manner, by employing two molar equivalents of oxidant.

Compounds of general formula I wherein Y represents the fluorine atom may be prepared by diazotisation of corresponding amines using sodium nitrite in tetrafluoroboric acid and sulphuric acid at a temperature from -10°C to +10°C, followed by photolysis in the presence of excess sodium tetrafluoroboric acid at a temperature from -30°C to ambient.

Intermediate amines above may be prepared by reduction of compounds of general formula I wherein Y is replaced by a nitro group, preferably with zinc in ethanol at a temperature from ambient to reflux.

Compounds of general formula I wherein Y represents the methyl group may be prepared by reduction of an acid of general formula XVI using a reducing agent, preferably borane-tetrahydrofuran complex in a solvent preferably tetrahydrofuran at a temperature from -30°C to reflux.

Compounds of general formula I wherein Z represents a trialkylsilylmethyl group as hereinbefore defined may be prepared by the reaction of a compound of general formula I wherein Z represents the methyl group with a lithiating agent preferably lithium diisopropylamide or n-butyl lithium, and reaction with a trialkylsilyl halide at a temperature from -78°C to ambient, and in an inert organic solvent preferably tetrahydrofuran, optionally in an inert atmosphere.

The following processes optionally followed by the subsidiary processes hereinbefore described permit the preparation of the remaining compounds of general formula I not described above, as well as some whose preparation is described above.

The diamino compounds above wherein Y represents the cyano group may be prepared by the reaction of potassium cyanoform KC(CN)₃ with a phenylhydrazine of general formula II in the presence of hydrochloric acid, at a temperature from 50°C to the reflux temperature of the reaction mixture.

Compounds of general formula I wherein Y represents a 1,1-difluoroalkyl group which may be substituted by one or more additional halogen atoms may be prepared by the reaction of a compound of general formula I wherein Y represents a straight- or branched-chain alkanoyl group containing from 2 to 6 carbon atoms or the corresponding compound in which Y is replaced by the formyl group or a straight- or branched-chain alkanoyl group containing from 2 to 6 carbon atoms which is substituted by one or more halogen atoms with a fluorinating agent, preferably diethylaminosulphur trifluoride or sulphur tetrafluoride in an inert organic solvent, preferably dichloromethane, at a temperature from -78°C to ambient.

Compounds of general formula I wherein Y represents the trifluoromethyl group or a trifluoromethylalkyl group containing from 2 to 6 carbon atoms which may be substituted by one or more additional halogen atoms may be prepared by the reaction of a fluorinating agent, e.g.sulphur tetrafluoride, with an acid of general formula XVI or the corresponding carboxyalkyl compound (it being understood that the carboxy group may be attached to any position of the alkyl moiety) at a temperature from ambient to 150°C.

Salts with pesticidally-acceptable bases of compounds of general formula I wherein Z represents the carboxy group may be prepared from the corresponding compounds of general formula I by methods known per se, for example by reacting stoichiometric quantities of the compound of general formula I and the appropriate base, for example an alkali metal hydroxide, carbonate or bicarbonate, an alkaline earth metal hydroxide or carbonate, ammonia or an amine (e.g. diethanolamine, triethanolamine, octylamine, morpholine or dioctylamine), in a suitable solvent. The salts may, if necessary, be purified by recrystallisation from one, two or more suitable solvents.

Compounds of general formula I not hitherto disclosed or described in the chemical literature, together with their processes of preparation form further features of the present invention.

The present invention accordingly provides the compounds of general formula I, wherein the various symbols are as hereinbefore defined, and salts thereof.

The following Example and Reference Example illustrate the preparation of compounds of general formula I according to the present invention:-

### REFERENCE EXAMPLE 1

The phenylhydrazine used as starting material in Example 1, not hitherto described in the chemical literature was prepared as follows:-
2,6-Dichloro-4-trifluoromethylphenylaniline (4.3 g) was dissolved with stirring, in glacial acetic acid (23 ml). A solution of sodium nitrite (1.5 g) in concentrated sulphuric acid (11 ml) was then added at 55-60°C. The solution thus obtained was cooled to 0-5°C and a solution of stannous chloride (16.4 g) in concentrated hydrochloric acid (14 ml) was added with vigorous stirring. A cream-coloured solid precipitated. The mixture was filtered and the solid obtained was added to a mixture of aqueous ammonia solution and ice. The mixture thus obtained was extracted with diethyl ether (6 x 500 ml) and the combined ethereal extracts were dried over sodium sulphate, filtered and evaporated to dryness to give 2,6-dichloro-4-trifluoromethylphenylhydrazine (3.7 g) m.p. 54-56°C, in the form of a colourless crystalline solid.

### EXAMPLE 1

### Compound No. 1

A solution of 2,6-dichloro-4-trifluoromethylphenylhydrazine (3.8 g) and 1,1-dicyano-2-cyclopropyl-2-methoxyethylene (2.23 g) in methanol (30 ml) was stirred and treated with sodium hydride (80%, 30 mg). After 4 hours the solution was evaporated in vacuo and the residue was dissolved in ethyl acetate (40 ml), treated with charcoal and washed with water. The organic phase was evaporated in vacuo, the residual oil was dissolved in petroleum ether and crystals of 5-amino-4-cyano- 3-cyclopropyl-1-(2,6-dichloro-4-trifluoromethylphenyl)pyrazole, m.p. 197-199°C, were obtained.
In the following formulae it is to be understood that:

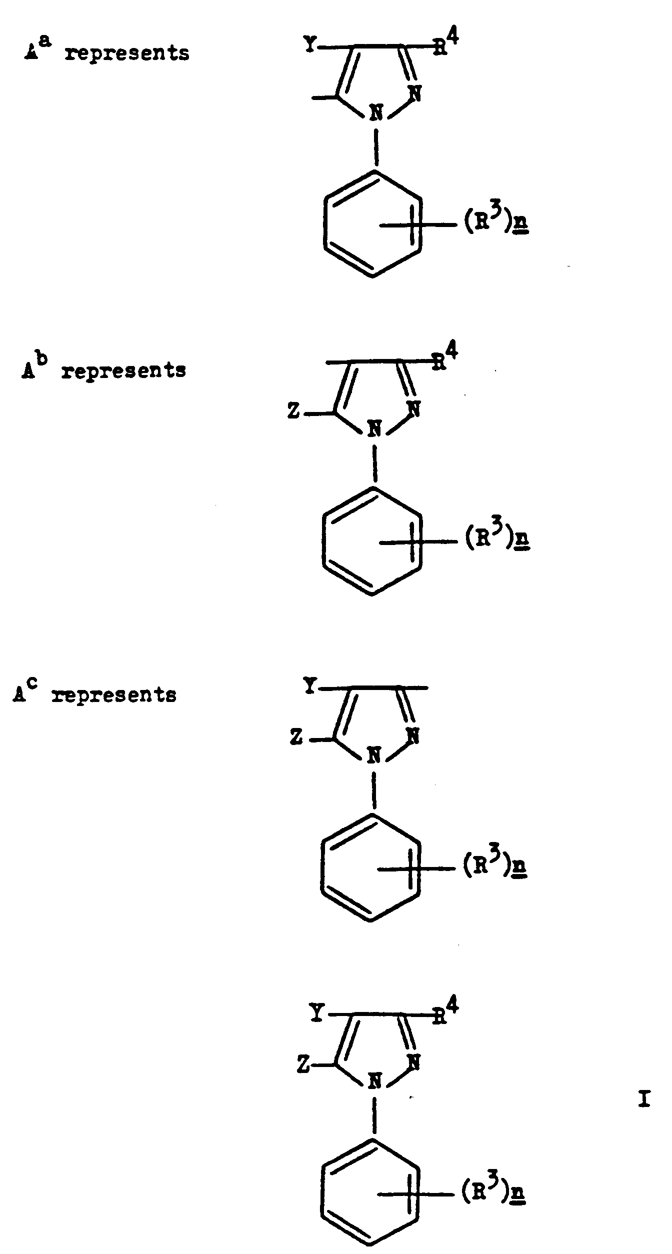

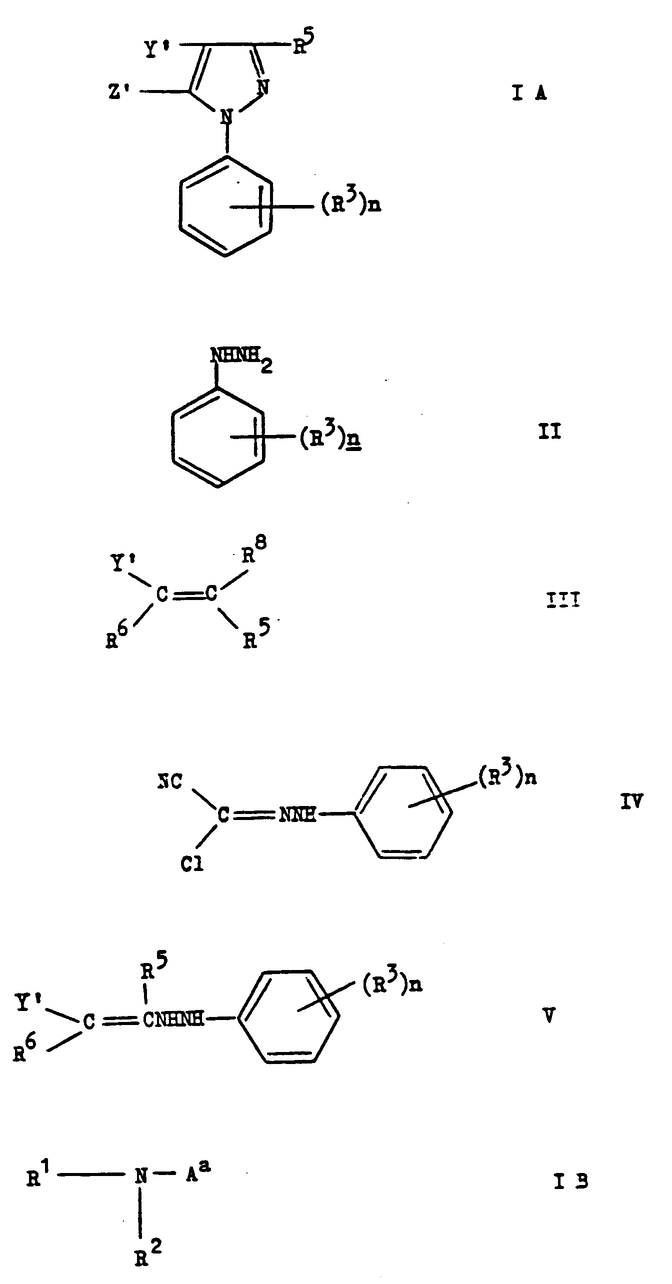

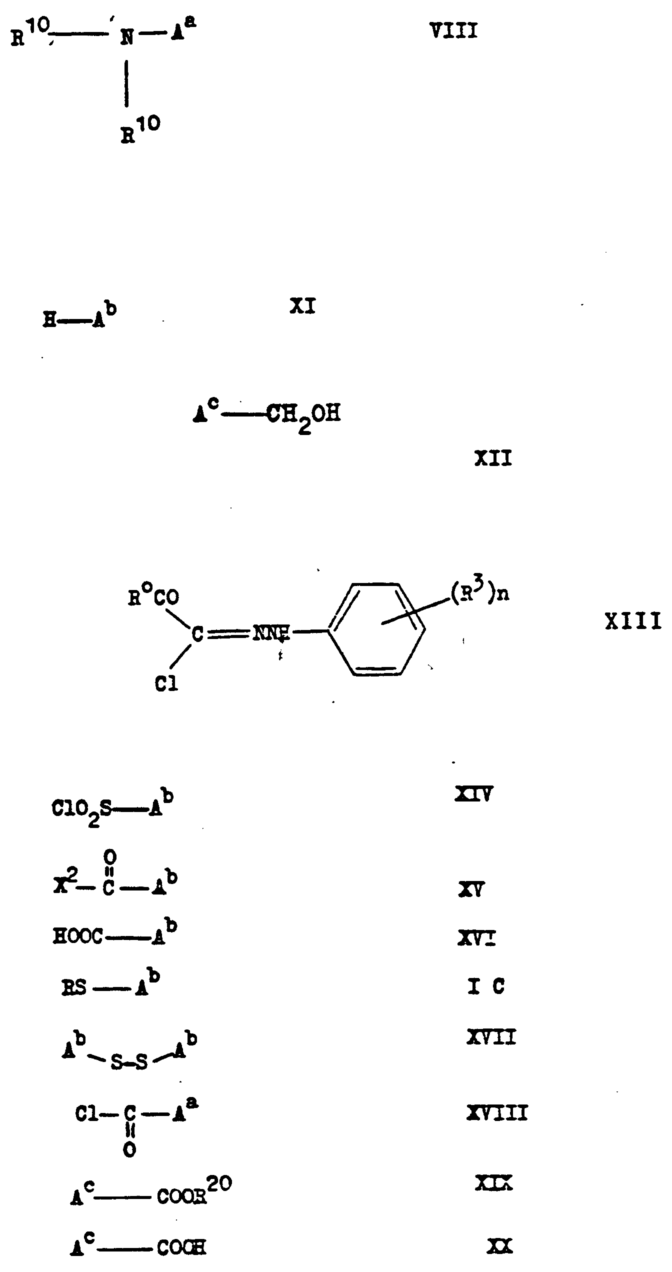

## Claims (Claims for the following Contracting State(s): BE, CH, DE, DK, FR, GB, GR, IT, LI, LU, NL, SE)

1. A method for the control of arthropod, plant nematode or helminth pests at a locus which comprises treatment of the locus with an effective amount of a compound of the general formula: 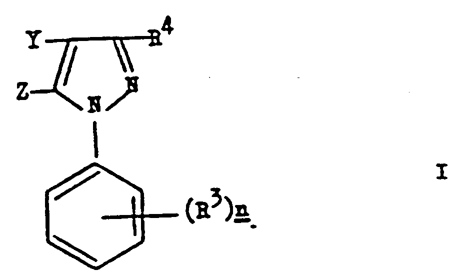 wherein Y represents a halogen atom, the cyano group or a group RSO₂, RSO or RS in which R represents a straight- or branched-chain alkyl group containing from 1 to 6 carbon atoms which is unsubstituted or substituted by one or more halogen atoms, a cycloalkyl group containing from 3 to 5 carbon atoms, a straight- or branched-chain alkenyl group containing from 2 to 6 carbon atoms, the thiocyanato group, the sulphamoyl group which is unsubstituted or substituted by one or two straight- or branched-chain alkyl groups which may be the same or different and contain from 1 to 6 atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms, a straight- or branched-chain alkanoyl group containing from 2 to 7 carbon atoms, or a straight- or branched-chain alkyl group containing from 1 to 6 carbon atoms which is unsubstituted or substituted by one or more halogen atoms, Z represents the hydrogen atom, or the amino group -NR¹R² wherein R¹ and R², which may be the same or different, each represents a hydrogen atom or a straight- or branched-chain alkyl group (containing from 1 to 6 carbon atoms, and which is unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contains from 2 to 7 carbon atoms or together form a 5 or 6 membered cyclic imide with the nitrogen atom to which they are attached and themselves are unsubstituted or substituted by one or more halogen atoms) or cycloalkylcarbonyl group (which contains from 4 to 7 carbon atoms) or straight- or branched-chain alkoxycarbonyl group (which contains from 2 to 7 carbon atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which is unsubstituted or substituted on methylene by a straight- or branched-chain alkyl group containing from 1 to 4 carbon atoms, or represents a halogen atom, a straight- or branched-chain alkyl group containing from 1 to 4 carbon atoms, the carboxy group, or a straight- or branched-chain alkylthio, alkylsulphinyl or alkylsulphonyl group containing from 1 to 6 carbon atoms which is unsubstituted or substituted by one or more halogen atoms, or represents a straight- or branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, R³ represents a halogen atom, a straight- or branched-chain alkyl or alkoxy group containing from 1 to 4 carbon atoms which is unsubstituted or substituted by one or more halogen atoms, a straight- or branched-chain alkylthio or alkylsulphinyl group containing from 1 to 4 carbon atoms which is substituted by one or more halogen atoms, the nitro or cyano group or a straight- or branched-chain alkylsulphonyl group containing from 1 to 4 carbon atoms which is unsubstituted or substituted by one or more halogen atoms, and R⁴ represents a cycloalkyl group containing from 3 to 6 carbon atoms, and n represents an integer from 1 to 5 inclusive, and, when Z represents a carboxy group, salts thereof with pesticidally-acceptable bases, with the exclusion of a method for the treatment of the human or animal body carried out by a medical or veterinary practitioner as therapy.

2. A method according to claim 1 wherein in general formula I (R³)ₙ represents 2,4,6-trichloro,
2,3,5,6-tetrachloro, 2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichloro-4-nitro,
2,6-dichloro-4-trifluoromethylsulphinyl,
2,6-dichloro-4-methanesulphonyl or
2,6-dichloro-4-trifluoromethanesulphonyl substitution.

3. A method according to claim 1 wherein in general formula I (R³)ₙ represents 2,6-dichloro-4-trifluoromethyl or 2,6-dichloro-4-trifluoromethoxy substitution.

4. An arthropodicidal, plant nematocidal or anthelmintic composition which comprises at least one compound of general formula I, or a pesticidally acceptable salt thereof, in association with one or more compatible diluents or carriers.

5. A compound of general formula I, wherein the various symbols are as defined in claim 1, and salts thereof.

6. A compound according to claim 5 wherein (R³)ₙ represents 2,4,6-trichloro, 2,3,5,6-tetrachloro,
2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichloro-4-nitro,
2,6-dichloro-4-trifluoromethylsulphinyl,
2,6-dichloro-4-methanesulphonyl or
2,6-dichloro-4-trifluoromethanesulphonyl substitution.

7. A compound according to claim 5 wherein (R³)ₙ represents 2,6-dichloro-4-trifluoromethyl or 2,6-dichloro-4-trifluoromethoxy substitution.

8. A process for the preparation of a compound of general formula I as defined in claim 1 which comprises when the compound of general formula I conforms to the general formula: 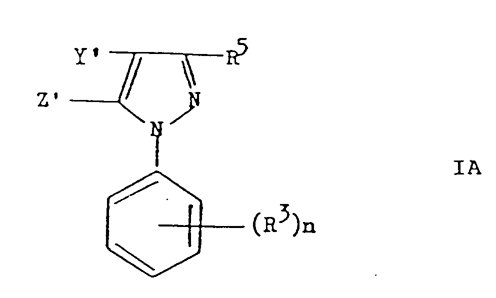 wherein Y' represents the cyano group or a group RSO₂, RSO or RS, wherein R is as defined in claim 1, a straight- or branched-chain alkoxycarbonyl group containing from 2 to 7 carbon atoms, or a straight- or branched-chain alkyl group containing form 1 to 6 carbon atoms which is unsubstituted or substituted by one or more halogen atoms, Z' represents the unsubstituted amino group or a straight- or branched-chain alkyl group containing from 1 to 4 carbon atoms, and R⁵ represents a cycloalkyl group containing from 3 to 6 carbon atoms:
(i) the reaction of a compound of the general formula: 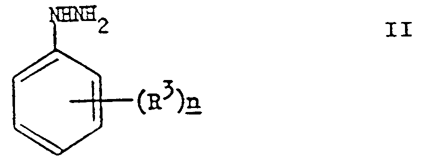 wherein R³ and n are as defined in claim 1, or an acid addition salt thereof with a compound of the general formula: 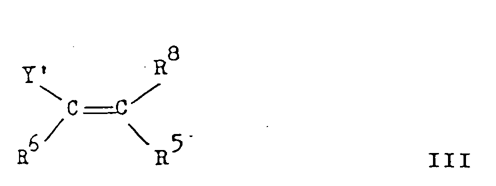 wherein Y' and R⁵ are as hereinbefore defined, R⁶ represents the cyano group or a straight- or branched-chain alkanoyl group containing from 2 to 5 carbon atoms and R⁸ represents a straight- or branched-chain alkoxy group containing from 1 to 4 carbon atoms, the hydroxy group or a fluorine, chlorine or bromine atom, or
(ii) when Z' in general formula IA represents the unsubstituted amino group, the reaction of a compound of general formula Y'CH₂CN with a compound of general formula II in the presence of a compound of the general formula R⁷C(R⁰)₃ wherein R⁷ represents a cycloalkyl group containing from 3 to 6 carbon atoms and R⁰ represents an alkoxy group in an inert organic solvent at a temperature from ambient to reflux; and preparing other compounds of general formula I by the conversion, as hereinbefore described, of one or more substituents Y, Z, R³ and R⁴, or substituents corresponding thereto, into substituents Y, Z, R³ and R⁴ as defined in claim 1; and, when Z represents the carboxy group, optionally converting a compound of general formula I into a salt thereof.

9. A compound of general formula I as defined in claim 1, or a pesticidally acceptable salt thereof, for use in the manufacture of a medicament for the treatment of an arthropod or helminth infection.

## Claims (Claims for the following Contracting State(s): AT, ES)

1. A process for the preparation of a compound of the general formula: 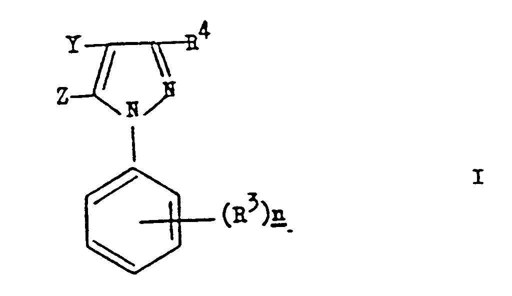 wherein Y represents a halogen atom, the cyano group or a group RSO₂, RSO or RS in which R represents a straight- or branched-chain alkyl group containing from 1 to 6 carbon atoms which is unsubstituted or substituted by one or more halogen atoms, a cycloalkyl group containing from 3 to 5 carbon atoms, a straight- or branched-chain alkenyl group containing from 2 to 6 carbon atoms, the thiocyanato group, the sulphamoyl group which is unsubstituted or substituted by one or two straight- or branched-chain alkyl groups which may be the same or different and contain from 1 to 6 atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms. a straight- or branched-chain alkanoyl group containing from 2 to 7 carbon atoms, or a straight- or branched-chain alkyl group containing from 1 to 6 carbon atoms which is unsubstituted or substituted by one or more halogen atoms, Z represents the hydrogen atom, or the amino group -NR¹R² wherein R¹ and R², which may be the same or different, each represents a hydrogen atom or a straight- or branched-chain alkyl group (containing from 1 to 6 carbon atoms, and which is unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contains from 2 to 7 carbon atoms or together form a 5 or 6 membered cyclic imide with the nitrogen atom to which they are attached and themselves are unsubstituted or substituted by one or more halogen atoms) or cycloalkylcarbonyl group (which contains from 4 to 7 carbon atoms) or straight- or branched-chain alkoxycarbonyl group (which contains from 2 to 7 carbon atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which is unsubstituted or substituted on methylene by a straight- or branched-chain alkyl group containing from 1 to 4 carbon atoms, or represents a halogen atom, a straight- or branched-chain alkyl group containing from 1 to 4 carbon atoms, the carboxy group, or a straight- or branched-chain alkylthio, alkylsulphinyl or alkylsulphonyl group containing from 1 to 6 carbon atoms which is unsubstituted or substituted by one or more halogen atoms, or represents a straight- or branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, R³ represents a halogen atom, a straight- or branched-chain alkyl or alkoxy group containing from 1 to 4 carbon atoms which is unsubstituted or substituted by one or more halogen atoms, a straight- or branched-chain alkylthio or alkylsulphinyl group containing from 1 to 4 carbon atoms which is substituted by one or more halogen atoms, the nitro or cyano group or a straight- or branched-chain alkylsulphonyl group containing from 1 to 4 carbon atoms which is unsubstituted or substituted by one or more halogen atoms, and R⁴ represents a cycloalkyl group containing from 3 to 6 carbon atoms, and n represents an integer from 1 to 5 inclusive, and, when Z represents a carboxy group, salts thereof with pesticidally-acceptable bases, which process comprises when the compound of general formula I conforms to the general formula: 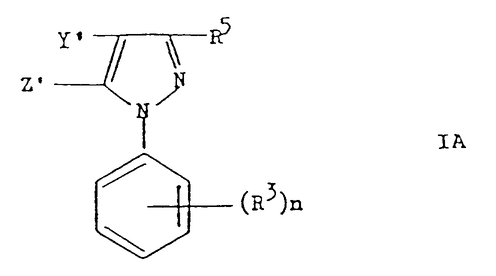 wherein Y' represents the cyano group or a group RSO₂, RSO or RS, wherein R is as hereinbefore defined, a straight- or branched-chain alkoxycarbonyl group containing from 2 to 7 carbon atoms, or a straight- or branched-chain alkyl group containing form 1 to 6 carbon atoms which is unsubstituted or substituted by one or more halogen atoms, Z' represents the unsubstituted amino group or a straight- or branched-chain alkyl group containing from 1 to 4 carbon atoms, and R⁵ represents a cycloalkyl group containing from 3 to 6 carbon atoms:
(i) the reaction of a compound of the general formula: 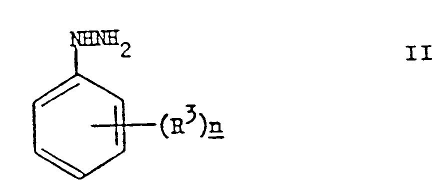 wherein R³ and n are as hereinbefore defined, or an acid addition salt thereof with a compound of the general formula: 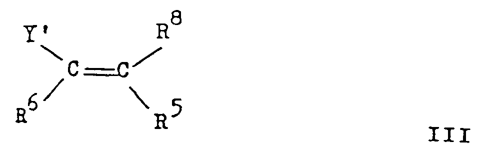 wherein Y' and R⁵ are as hereinbefore defined, R⁶ represents the cyano group or a straight- or branched-chain alkanoyl group containing from 2 to 5 carbon atoms and R⁸ represents a straight- or branched-chain alkoxy group containing from 1 to 4 carbon atoms, the hydroxy group or a fluorine, chlorine or bromine atom, or
(ii) when Z' in general formula IA represents the unsubstituted amino group, the reaction of a compound of general formula Y'CH₂CN with a compound of general formula II in the presence of a compound of the general formula R⁷C(R⁰)₃ wherein R⁷ represents a cycloalkyl group containing from 3 to 6 carbon atoms and R⁰ represents an alkoxy group in an inert organic solvent at a temperature from ambient to reflux; and preparing other compounds of general formula I by the conversion, as hereinbefore described, of one or more substituents Y, Z, R³ and R⁴, or substituents corresponding thereto, into substituents Y, Z, R³ and R⁴ as hereinbefore defined; and, when Z represents the carboxy group, optionally converting a compound of general formula I into a salt thereof.

2. A process according to claim 1 wherein in general formula I (R³)ₙ represents 2,4,6-trichloro,
2,3,5,6-tetrachloro, 2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichloro-4-nitro,
2,6-dichloro-4-trifluoromethylsulphinyl,
2,6-dichloro-4-methanesulphonyl or
2,6-dichloro-4-trifluoromethanesulphonyl substitution.

3. A process according to claim 1 wherein in general formula I (R³)ₙ represents 2,6-dichloro-4-trifluoromethyl or 2,6-dichloro-4-trifluoromethoxy substitution.

4. A process for the preparation of an arthropodicidal, plant nematocidal or anthelmintic composition which comprises formulating at least one compound of general formula I as defined in claim 1, or a pesticidally acceptable salt thereof, with one or more compatible diluents or carriers.

5. A method for the control of arthropod, plant nematode or helminth pests at a locus which comprises treatment of the locus with an effective amount of a compound of general formula I as defined in claim 1, or a salt thereof, with the exclusion of a method for the treatment of the human or animal body carried out by a medical or veterinary practitioner as therapy.

6. A method according to claim 5 wherein (R³)ₙ represents 2,4,6-trichloro, 2,3,5,6-tetrachloro,
2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichlor-4-nitro,
2,6-dichloro-4-trifluoromethylsulphinyl,
2,6-dichloro-4-methanesulphonyl or
2,6-dichloro-4-trifluoromethanesulphonyl substitution.

7. A method according to claim 5 wherein (R³)ₙ represents 2,6-dichloro-4-trifluoromethyl or 2,6-dichloro-4-trifluoromethoxy substitution.

## Patentansprüche (Patentansprüche für folgende(n) Vertragsstaat(en): BE, CH, DE, DK, FR, GB, GR, IT, LI, LU, NL, SE)

1. Verfahren zur lokalen Bekämpfung von schädlichen Arthropoden, Pflanzennematoden oder Helminthen, das die Behandlung des betreffenden Orts mit einer wirksamen Menge einer Verbindung der allgemeinen Formel 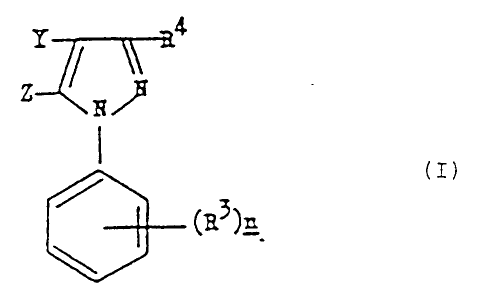 umfaßt, in der bedeuten:
- Y ein Halogen, Cyano oder eine Gruppe RSO₂, RSO oder RS, worin R eine geradkettige oder verzweigte C₁₋₆-Alkylgruppe, die ggf. mit einem oder mehreren Halogenatomen substituiert ist, C₃₋₅-Cycloalkyl oder geradkettiges oder verzweigtes C₂₋₆-Alkenyl darstellt, Thiocyanato, eine Sulfamoylgruppe, die ggf. mit ein oder zwei geradkettigen oder verzweigten C₁₋₆-Alkylgruppen substituiert ist, die gleich oder verschieden sein können, eine Carbamoylgruppe, die ggf. mit einer oder zwei geradkettigen oder verzweigten C₁₋₆-Alkylgruppen substituiert ist, die gleich oder verschieden sein können, eine geradkettige oder verzweigte Alkoxycarbonylgruppe mit 2 bis 7 Kohlenstoffatomen, eine geradkettige oder verzweigte Alkanoylgruppe mit 2 bis 7 Kohlenstoffatomen oder eine geradkettige oder verzweigte C₁₋₆-Alkylgruppe, die ggf. mit ein oder mehreren Halogenatomen substituiert ist,
- Z Wasserstoff oder eine Aminogruppe -NR¹R², worin R¹ und R², die gleich oder verschieden sein können, unabhängig Wasserstoff, eine geradkettige oder verzweigte Alkylgruppe (die 1 bis 6 Kohlenstoffe enthält und ggf. mit einer geradkettigen oder verzweigten C₂₋₅-Alkoxycarbonylgruppe substituiert ist), C₃₋₆-Cycloalkyl, Formyl, eine geradkettige oder verzweigte Alkanoylgruppe (die 2 bis 7 Kohlenstoffatome enthält oder die zusammen mit dem Stickstoffatom, an das sie gebunden sind, ein 5- oder 6gliedriges ringförmiges Imid bilden, und die selbst ggf. mit einem oder mehreren Halogenatomen substituiert sind), eine Cycloalkylcarbonylgruppe (die 4 bis 7 Kohlenstoffatome enthält) oder eine geradkettige oder verzweigte Alkoxycarbonylgruppe (die 2 bis 7 Kohlenstoffatome enthält und die ggf. selbst mit einem oder mehreren Halogenatomen substituiert sind) darstellen,
Z weiterhin eine geradkettige oder verzweigte Alkylsulfenylaminogruppe mit 1 bis 4 Kohlenstoffatomen, eine geradkettige oder verzweigte Alkoxymethylenaminogruppe mit 2 bis 5 Kohlenstoffatomen, wobei der Methylenrest ggf. mit einer geradkettigen oder verzweigten C₁₋₄-Alkylgruppe substituiert ist, oder
ein Halogen, geradkettiges oder verzweigtes C₁₋₄-Alkyl, Carboxy, geradkettiges oder verzweigtes Alkylthio, Alkylsulfenyl oder Alkylsulfonyl mit 1 bis 6 Kohlenstoffatomen, das ggf. mit einem oder mehreren Halogenatomen substituiert ist, oder
geradkettiges oder verzweigtes Trialkylsilylmethyl mit 1 bis 6 Kohlenstoffatomen in den Alkylgruppen, die gleich oder verschieden sein können, Trialkylsilyl mit 1 bis 6 Kohlenstoffatomen in den Alkylgruppen, die gleich oder verschieden sein können, Cyano oder Nitro,
- R³ ein Halogen, eine geradkettige oder verzweigte Alkyl- oder Alkoxygruppe mit 1 bis 4 Kohlenstoffatomen, die ggf. mit einem oder mehreren Halogenatomen substituiert ist, eine geradkettige oder verzweigte Alkylthio- oder Alkylsulfinylgruppe mit 1 bis 4 Kohlenstoffatomen, die ggf. mit einem oder mehreren Halogenatomen substituiert ist, Nitro oder Cyano, oder eine geradkettige oder verzweigte Alkylsulfonylgruppe mit 1 bis 4 Kohlenstoffatomen, die ggf. mit einem oder mehreren Halogenatomen substituiert ist, und
- R⁴ C₃₋₆-Cycloalkyl,
- n eine ganze Zahl von 1 bis 5 einschließlich,
einschließlich der Salze mit für Pestizide akzeptablen Basen, wenn Z eine Carboxygruppe darstellt, wobei Verfahren zur therapeutischen Behandlung des menschlichen oder tierischen Körpers, die von einem Arzt oder einem Veterinärmediziner durchgeführt werden, ausgeschlossen sind.

2. Verfahren nach Anspruch 1, wobei (R³)ₙ in der allgemeinen Formel I eine 2,4,6-Trichlor-, 2,3,5,6-Tetrachlor-, 2-Chlor-4-trifluormethyl-, 2,3,5,6-Tetrafluor-4-trifluormethyl-, 2,6-Dichlor-4-trifluormethylthio-, 2-Chlor-3,5,6-trifluor-4-trifluormethyl-, 2,6-Dichlor-3,5-difluor-4-trifluormethyl-, 2,6-Dichlor-4-nitro-, 2,6-Dichlor-4-trifluormethylsulfinyl-, 2,6-Dichlor-4-methansulfonyl- oder 2,6-Dichlor-4-trifluormethansulfonyl-Substitution darstellt.

3. Verfahren nach Anspruch 1, wobei (R³)ₙ in der allgemeinen Formel I eine 2,6-Dichlor-4-trifluormethyl- oder 2,6-Dichlor-4-trifluormethoxy-Substitution darstellt.

4. Arthropodizide, pflanzennematozide oder anthelmintische Zusammensetzungen, die eine oder mehrere Verbindungen der allgemeinen Formel I oder ein für Pestizide akzeptables Salz davon in Kombination mit einem oder mehreren verträglichen Verdünnungsmitteln oder Trägern enthalten.

5. Verbindungen der allgemeinen Formel I, wobei die verschiedenen Symbole wie in Anspruch 1 definiert sind, und Salze davon.

6. Verbindungen nach Anspruch 1, wobei (R³)ₙ eine 2,4,6-Trichlor-, 2,3,5,6-Tetrachlor-, 2-Chlor-4-trifluormethyl-, 2,3,5,6-Tetrafluor-4-trifluormethyl-, 2,6-Dichlor-4-trifluormethylthio-, 2-Chlor-3,5,6-trifluor-4-trifluormethyl-, 2,6-Dichlor-3,5-difluor-4-trifluormethyl-, 2,6-Dichlor-4-nitro-, 2,6-Dichlor-4-trifluormethylsulfinyl-, 2,6-Dichlor-4-methansulfonyl- oder 2,6-Dichlor-4-trifluormethansulfonyl-Substitution darstellt.

7. Verbindungen nach Anspruch 5, wobei (R³)ₙ eine 2,5-Dichlor-4-trifluormethyl- oder 2,6-Dichlor-4-trifluormethoxy-Substitution darstellt.

8. Verfahren zur Herstellung von Verbindungen der allgemeinen Formel I gemäß Anspruch 1, das, wenn die Verbindung der allgemeinen Formel I der allgemeinen Formel 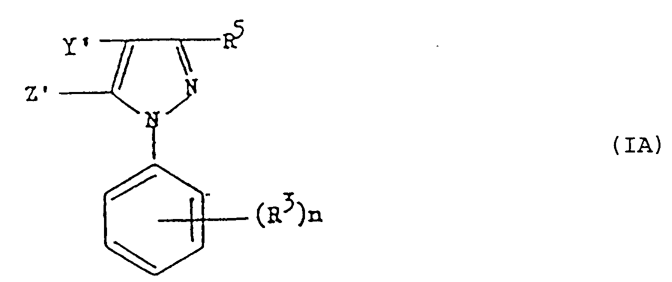 entspricht, in der bedeuten:
- Y' Cyano oder eine Gruppe RSO₂, RSO oder RS, worin R wie in Anspruch 1 definiert ist, eine geradkettige oder verzweigte Alkoxycarbonylgruppe mit 2 bis 7 Kohlenstoffatomen, eine geradkettige oder verzweigte C₁₋₆-Alkylgruppe, die ggf. mit einem oder mehreren Halogenatomen substituiert ist,
- Z' die unsubstituierte Aminogruppe oder eine geradkettige oder verzweigte C₁₋₄-Alkylgruppe und
- R⁵ eine C₃₋₆-Cycloalkylgruppe,
folgende Schritte umfaßt:
(i) Umsetzung einer Verbindung der allgemeinen Formel 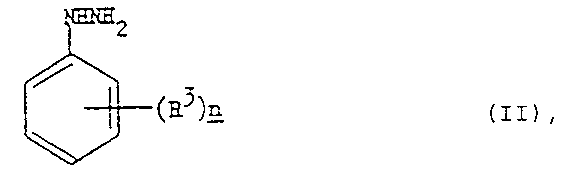 worin R³ und n wie in Anspruch 1 definiert sind, oder eines Säureadditionssalzes davon mit einer Verbindung der allgemeinen Formel 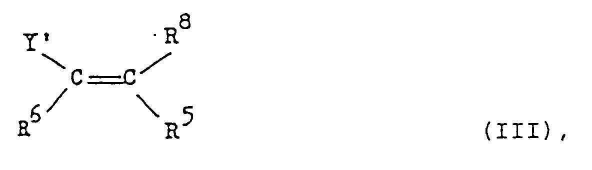 worin Y' und R⁵ wie oben definiert sind, R⁶ Cyano oder eine geradkettige oder verzweigte Alkanoylgruppe mit 2 bis 5 Kohlenstoffatomen und R⁸ eine geradkettige oder verzweigte C₁₋₄-Alkoxygruppe, Hydroxy, Fluor, Chlor oder Brom darstellen, oder
(ii) wenn Z' in der allgemeinen Formel IA eine unsubstituierte Aminogruppe ist, Umsetzung einer Verbindung der allgemeinen Formel Y'CH₂CN mit einer Verbindung der allgemeinen Formel II in Gegenwart einer Verbindung der allgemeinen Formel R⁷C(R⁰)₃, worin R⁷ eine C₃₋₆-Cycloalkylgruppe und R⁰ eine Alkoxygruppe darstellen, in einem inerten organischen Lösungsmittel bei einer Temperatur von Umgebungstemperatur bis zur Rückflußtemperatur, und
Herstellung anderer Verbindungen der allgemeinen Formel I durch die Umwandlung, wie oben beschrieben, von einem oder mehreren Substituenten Y, Z, R³ und R⁴ oder entsprechenden Substituenten in Substituenten Y, Z, R³, R⁴, wie sie in Anspruch 1 definiert sind, und wenn Z eine Carboxygruppe ist, wahlweise Umwandlung der Verbindungen der allgemeinen Formel I in Salze davon.

9. Verbindungen der allgemeinen Formel I gemäß Anspruch 1 und ihre für Pestizide akzeptablen Salze zur Behandlung des Befalls mit Arthropoden oder Helminthen.

## Patentansprüche (Patentansprüche für folgende(n) Vertragsstaat(en): AT, ES)

1. Verfahren zur Herstellung von Verbindungen der allgemeinen Formel 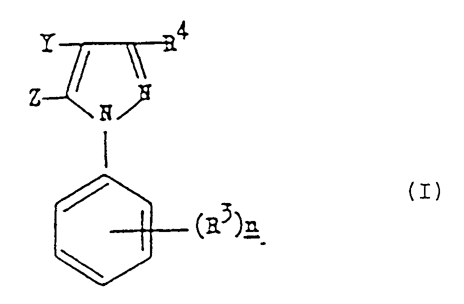 umfaßt, in der bedeuten:
- Y ein Halogen, Cyano oder eine Gruppe RSO₂, RSO oder RS, worin R eine geradkettige oder verzweigte C₁₋₆-Alkylgruppe, die ggf. mit einem oder mehreren Halogenatomen substituiert ist, C₃₋₅-Cycloalkyl oder geradkettiges oder verzweigtes C₂₋₆-Alkenyl darstellt, Thiocyanato, eine Sulfamoylgruppe, die ggf. mit ein oder zwei geradkettigen oder verzweigten C₁₋₆-Alkylgruppen substituiert ist, die gleich oder verschieden sein können, eine Carbamoylgruppe, die ggf. mit einer oder zwei geradkettigen oder verzweigten C₁₋₆-Alkylgruppen substituiert ist, die gleich oder verschieden sein können, eine geradkettige oder verzweigte Alkoxycarbonylgruppe mit 2 bis 7 Kohlenstoffatomen, eine geradkettige oder verzweigte Alkanoylgruppe mit 2 bis 7 Kohlenstoffatomen oder eine geradkettige oder verzweigte C₁₋₆-Alkylgruppe, die ggf. mit ein oder mehreren Halogenatomen substituiert ist,
- Z Wasserstoff oder eine Aminogruppe -NR¹R², worin R¹ und R², die gleich oder verschieden sein können, unabhängig Wasserstoff, eine geradkettige oder verzweigte Alkylgruppe (die 1 bis 6 Kohlenstoffe enthält und ggf. mit einer geradkettigen oder verzweigten C₂₋₅-Alkoxycarbonylgruppe substituiert ist), C₃₋₆-Cycloalkyl, Formyl, eine geradkettige oder verzweigte Alkanoylgruppe (die 2 bis 7 Kohlenstoffatome enthält oder die zusammen mit dem Stickstoffatom, an das sie gebunden sind, ein 5- oder 6gliedriges ringförmiges Imid bilden, und die selbst ggf. mit einem oder mehreren Halogenatomen substituiert sind), eine Cycloalkylcarbonylgruppe (die 4 bis 7 Kohlenstoffatome enthält) oder eine geradkettige oder verzweigte Alkoxycarbonylgruppe (die 2 bis 7 Kohlenstoffatome enthält und die ggf. selbst mit einem oder mehreren Halogenatomen substituiert sind) darstellen,
Z weiterhin eine geradkettige oder verzweigte Alkylsulfenylaminogruppe mit 1 bis 4 Kohlenstoffatomen, eine geradkettige oder verzweigte Alkoxymethylenaminogruppe mit 2 bis 5 Kohlenstoffatomen, wobei der Methylenrest ggf. mit einer geradkettigen oder verzweigten C₁₋₄-Alkylgruppe substituiert ist, oder
ein Halogen, geradkettiges oder verzweigtes C₁₋₄-Alkyl, Carboxy, geradkettiges oder verzweigtes Alkylthio, Alkylsulfenyl oder Alkylsulfonyl mit 1 bis 6 Kohlenstoffatomen, das ggf. mit einem oder mehreren Halogenatomen substituiert ist, oder
geradkettiges oder verzweigtes Trialkylsilylmethyl mit 1 bis 6 Kohlenstoffatomen in den Alkylgruppen, die gleich oder verschieden sein können, Trialkylsilyl mit 1 bis 6 Kohlenstoffatomen in den Alkylgruppen, die gleich oder verschieden sein können, Cyano oder Nitro,
- R³ ein Halogen, eine geradkettige oder verzweigte Alkyl- oder Alkoxygruppe mit 1 bis 4 Kohlenstoffatomen, die ggf. mit einem oder mehreren Halogenatomen substituiert ist, eine geradkettige oder verzweigte Alkylthio- oder Alkylsulfinylgruppe mit 1 bis 4 Kohlenstoffatomen, die ggf. mit einem oder mehreren Halogenatomen substituiert ist, Nitro oder Cyano, oder eine geradkettige oder verzweigte Alkylsulfonylgruppe mit 1 bis 4 Kohlenstoffatomen, die ggf. mit einem oder mehreren Halogenatomen substituiert ist, und
- R⁴ C₃₋₆-Cycloalkyl,
- n eine ganze Zahl von 1 bis 5 einschließlich,
einschließlich der Salze mit für Pestizide akzeptablen Basen, wenn Z eine Carboxygruppe darstellt, das, wenn die Verbindung der allgemeinen Formel I der allgemeinen Formel 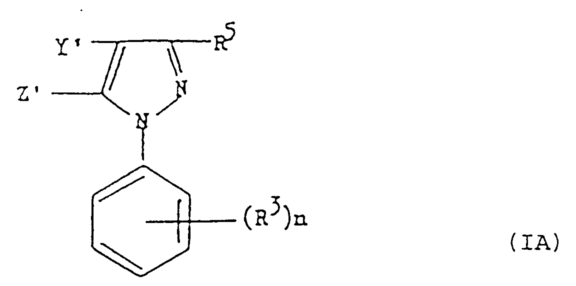 entspricht, in der bedeuten:
- Y' Cyano oder eine Gruppe RSO₂, RSO oder RS, worin R wie in Anspruch 1 definiert ist, eine geradkettige oder verzweigte Alkoxycarbonylgruppe mit 2 bis 7 Kohlenstoffatomen, eine geradkettige oder verzweigte C₁₋₆-Alkylgruppe, die ggf. mit einem oder mehreren Halogenatomen substituiert ist,
- Z' die unsubstituierte Aminogruppe oder eine geradkettige oder verzweigte C₁₋₄-Alkylgruppe und
- R⁵ eine C₃₋₆-Cycloalkylgruppe,
folgende Schritte umfaßt:
(i) Umsetzung einer Verbindung der allgemeinen Formel 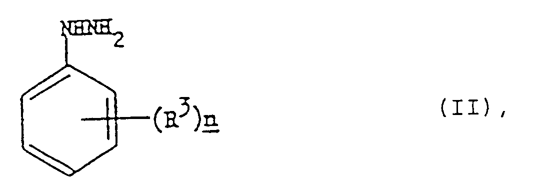 worin R³ und n wie in Anspruch 1 definiert sind, oder eines Säureadditionssalzes davon mit einer Verbindung der allgemeinen Formel 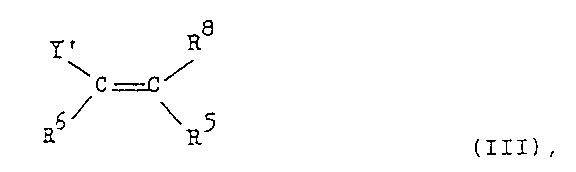 worin Y' und R⁵ wie oben definiert sind, R⁶ Cyano oder eine geradkettige oder verzweigte Alkanoylgruppe mit 2 bis 5 Kohlenstoffatomen und R⁸ eine geradkettige oder verzweigte C₁₋₄-Alkoxygruppe, Hydroxy, Fluor, Chlor oder Brom darstellen, oder
(ii) wenn Z' in der allgemeinen Formel IA die unsubstituierte Aminogruppe ist, Umsetzung einer Verbindung der allgemeinen Formel Y'CH₂CN mit einer Verbindung der allgemeinen Formel II in Gegenwart einer Verbindung der allgemeinen Formel R⁷C(R⁰)₃, worin R⁷ eine C₃₋₆-Cycloalkylgruppe und R⁰ eine Alkoxygruppe darstellen, in einem inerten organischen Lösungsmittel bei einer Temperatur von Umgebungstemperatur bis zur Rückflußtemperatur, und
Herstellung anderer Verbindungen der allgemeinen Formel I durch die Umwandlung, wie sie weiter oben beschrieben ist, von einem oder mehreren Substituenten Y, Z, R³ und R⁴ oder entsprechenden Substituenten in Substituenten Y, Z, R³, R⁴, wie sie in Anspruch 1 definiert sind, und wenn Z eine Carboxygruppe ist, wahlweise Umwandlung der Verbindungen der allgemeinen Formel I in Salze davon.

2. Verfahren nach Anspruch 1, wobei (R³)ₙ in der allgemeinen Formel I eine 2,4,6-Trichlor-, 2,3,5,6-Tetrachlor-, 2-Chlor-4-trifluormethyl-, 2,3,5,6-Tetrafluor-4-trifluormethyl-, 2,6-Dichlor-4-trifluormethylthio-, 2-Chlor-3,5,6-trifluor-4-trifluormethyl-, 2,6-Dichlor-3,5-difluor-4-trifluormethyl-, 2,6-Dichlor-4-nitro-, 2,6-Dichlor-4-trifluormethylsulfinyl-, 2,6-Dichlor-4-methansulfonyl- oder 2,6-Dichlor-4-trifluormethansulfonyl-Substitution darstellt.

3. Verfahren nach Anspruch 1, wobei (R³)ₙ in der allgemeinen Formel I eine 2,6-Dichlor-4-trifluormethyl- oder 2,6-Dichlor-4-trifluormethoxy-Substitution darstellt.

4. Verfahren zur Herstellung von arthropodiziden, pflanzennematoziden oder anthelmintischen Zusammensetzungen, das die Formulierung von einer oder mehreren Verbindungen der allgemeinen Formel I gemäß Anspruch 1 oder eines für Pestizide akzeptablen Salzes davon mit einem oder mehreren verträglichen Verdünnungsmitteln oder Trägern umfaßt.

5. Verfahren zur lokalen Bekämpfung von schädlichen Arthropoden, Pflanzennematoden oder Helminthen, das die Behandlung des betreffenden Ortes mit einer wirksamen Menge einer Verbindung der allgemeinen Formel I gemäß Anspruch 1 oder einem Salz davon umfaßt, wobei Verfahren zur therapeutischen Behandlung des menschlichen oder tierischen Körpers, die von einem Arzt oder einem Veterinärmediziner durchgeführt werden, ausgeschlossen sind.

6. Verfahren nach Anspruch 1, wobei (R³)ₙ in der allgemeinen Formel I eine 2,4,6-Trichlor-, 2,3,5,6-Tetrachlor-, 2-Chlor-4-trifluormethyl-, 2,3,5,6-Tetrafluor-4-trifluormethyl-, 2,6-Dichlor-4-trifluormethylthio-, 2-Chlor-3,5,6-trifluor-4-trifluormethyl-, 2,6-Dichlor-3,5-difluor-4-trifluormethyl-, 2,6-Dichlor-4-nitro-, 2,6-Dichlor-4-trifluormethylsulfinyl-, 2,6-Dichlor-4-methansulfonyl- oder 2,6-Dichlor-4-trifluormethansulfonyl-Substitution darstellt.

7. Verfahren nach Anspruch 1, wobei (R³)ₙ in der allgemeinen Formel I eine 2,6-Dichlor-4-trifluormethyl- oder 2,6-Dichlor-4-trifluormethoxy-Substitution darstellt.

## Revendications (Revendications pour l'(les) Etat(s) contractant(s) suivant(s): BE, CH, DE, DK, FR, GB, GR, IT, LI, LU, NL, SE)

1. Un procédé de contrôle des arthropodes, des nématodes des plantes ou des helminthes parasites en un lieu qui comprend le traitement du dit lieu avec une quantité efficace d'un composé de formule générale 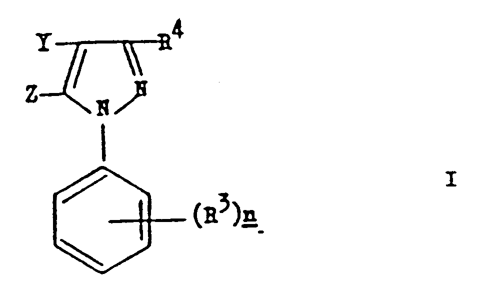 dans laquelle Y représente:un atome d'halogène, un radical cyano, ou un radical RSO₂, RSO, ou RS, dans lesquels R représente un radical alkyle, linéaire ou ramifié, contenant de 1 à 6 atomes de carbone, éventuellement substitué par un ou plusieurs atomes d'halogènes, un radical cycloalkyle contenant de 3 à 5 atomes de carbone, un radical alkényle, linéaire ou ramifié, contenant de 2 à 6 atomes de carbone, le radical thiocyanato,ou le radical sulfamoyle substitué ou non par un ou deux radicaux alkyle linéaires ou ramifiés, identiques ou différents qui peuvent contenir de 1 à 6 atomes de carbone, le radical carbamoyle, qui peut être substitué ou non par un ou deux radicaux alkyle, linéaires ou ramifiés, qui peuvent être identiques ou différents et contenir de 1 à 6 atomes de carbone, un radical alkoxycarbonyle contenant de 2 à 7 atomes de carbone, un radical alkanoyl, linéaire ou ramifié, contenant de 2 à 7 atomes de carbone, ou un radical alkyl, linéaire ou ramifié, contenant de 1 à 6 atomes de carbone, substitué ou non par un ou plusieurs atomes d'halogènes, Z représente: l'atome d'hydrogène, ou le radical amino - NR¹R² dans lequel R¹ et R², qui peuvent être identiques ou différents, représentent chacun un atome d'hydrogène, ou un radical alkyl, linéaire ou ramifié, (contenant de 1 à 6 atomes de carbone, et qui est substitué ou non par un ou plusieurs radicaux alkoxycarbonyl, linéaires ou ramifiés, contenant de 2 à 5 atomes de carbone), ou un radical cycloalkyl contenant de 3 à 6 atomes de carbone ou, un radical formyl, un radical alkanoyl linéaire ou ramifié (pouvant contenir de 2 à 7 atomes de carbone, ou former une imide cyclique à 5 ou 6 chaînons avec l'atome d'azote auquel ils sont reliés, eux-mêmes étant substitués ou non substitués par un ou plusieurs atomes d'halogènes), ou un radical cycloalkylcarbonyle, linéaire ou ramifié, (qui contient de 4 à 7 atomes de carbone) ou un radical alkoxycarbonyle, linéaire ou ramifié (qui contient de 2 à 7 atomes de carbone et sont eux-mêmes substitués par un ou plusieurs atomes d'halogènes), ou Z représente un radical alkylsulfénylamino, linéaire ou ramifié, contenant de 1 à 4 atomes de carbone, ou un radical alkoxyméthylèneamino, linéaire ou ramifié, contenant de 2 à 5 atomes de carbone, substitué ou non sur le radical méthylène par un radical alkyl, linéaire ou ramifié, contenant de 1 à 4 atomes de carbone, ou représente un atome d'halogène, un radical alkyl, linéaire ou ramifié, contenant de 1 à 4 atomes de carbone, ou le radical carboxy, ou un radical alkylthio, alkylsulfinyl ou alkylsulfonyl, linéaire ou ramifié, pouvant contenir de 1 à 6 atomes de carbone, et qui peut être substitué ou non par un ou plusieurs atomes d'halogènes, ou représente un radical trialkylsilylméthyl, linéaire ou ramifié, contenant de 1 à 6 atomes de carbone dans chaque radical alkyle qui peut être identique ou différent, ou un radical trialkylsilyl contenant de 1 à 6 atomes de carbone dans chaque radical alkyle qui peut être identique ou différent, ou le radical nitro ou le radical cyano; R³ représente un atome d'halogène, un radical alkyle ou alkoxy, linéaire ou ramifié, contenant de 1 à 4 atomes de carbone, substitué ou non substitué par un ou plusieurs atomes d'halogènes, un radical thioalkyle ou thiosulphinyl, linéaire ou ramifié, contenant de 1 à 4 atomes de carbone, et qui est substitué par un ou plusieurs atomes d'halogènes, et R⁴ représente un radical cycloalkyle contenant de 3 à 6 atomes de carbone, et n représente un nombre entier pouvant prendre toutes les valeurs de 1 à 5, et, quand Z représente un radical carboxy, les sels de ces composés obtenus avec des bases acceptables pour donner des pesticides, à l'exclusion d'une méthode de traitement des êtres humains ou des animaux réalisés à titre thérapeutique par des médecins ou des vétérinaires.

2. Un procédé selon la revendication 1 dans lequel, dans la formule générale I, (R³)ₙ représente :
2,4,6-trichloro,
2,3,5,6-tétrachloro,
2-chloro-4-trifluorométhyle,
2,3,5,6-tétrafluoro-4-trifluorométhyle,
2,6-dichloro-4-trifluorométhylthio,
2-chloro-3,5,6-trifluoro-4-trifluorométhyle,
2,6-dichloro-3,5-difluoro-4-trifluorométhyle,
2,6-dichloro-4-nitro,
2,6-dichloro-4-triflurométhylsulfinyle,
2,6-dichloro-4-méthanesulfonyle ou
2,6-dichloro-4-trifluorométhanesulfonyle.

3. Un procédé selon la revendication 1 dans lequel, dans la formule générale I, (R³)ₙ représente une substitution 2,6-dichloro-4-trifluorométhyle ou 2,6-dichloro-4-trifluorométhoxy.

4. Une composition arthropocide, nématocide pour les plantes ou anthelminthique qui contient au moins un composé de formule générale I, ou un sel de ces composés acceptable pour des compositions pesticides, en association avec un ou plusieurs charges ou diluants compatibles.

5. Un composé de formule générale I dans lequel les différents symboles ont la définition donnée dans la revendication I, et les sels de ces composés.

6. Un composé selon la revendication 5 dans lequel (R³)ₙ représente une substitution :
2,4,6-trichloro,
2,3,5,6-tétrachloro,
2-chloro-4-trifluorométhyle,
2,3,5,6-tétrafluoro-4-trifluorométhyle,
2,6-dichloro-4-trifluorométhylthio,
2-chloro-3,5,6-trifluoro-4-trifluorométhyle,
2,6-dichloro-3,5-difluoro-4-trifluorométhyle,
2,6-dichloro-4-nitro,
2,6-dichloro-4-triflurométhylsulfinyle,
2,6-dichloro-4-méthanesulfonyle ou
2,6-dichloro-4-trifluorométhanesulfonyle.

7. Un composé selon la revendication 5 dans lequel (R³)ₙ représente une substitution 2,6-dichloro-4-trifluorométhyl ou 2,6-dichloro-4-trifluorométhoxy.

8. Un procédé pour la préparation des composés de formule générale I tels que définis dans la revendication 1 qui comprend, lorsque les composés de formule générale I répondent à la formule générale 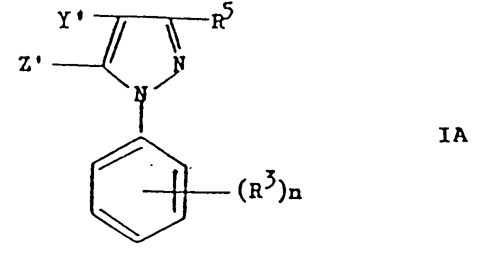 dans laquelle Y' représente un radical cyano ou un radical RSO₂, RSO ou RS ou un radical alkoxycarbonyle linéaire ou ramifié contenant de 2 à 7 atomes de carbone, ou un radical alkyle linéaire ou ramifié contenant de 1 à 6 atomes de carbone qui peuvent être substitués ou non substitués par un ou plusieurs atomes d'halogène, Z' représente un radical amino non substitué ou un radical alkyle linéaire ou ramifié contenant de 1 à 4 atomes de carbone, et R⁵ représente un radical cycloalkyle contenant de 3 à 6 atomes de carbone
(i) la réaction d'un composé de formule générale II 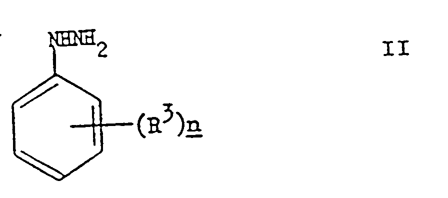 dans laquelle R³ et n ont la définition donnée dans la revendication 1, ou de l'un de ses sels, avec un composé de formule générale III 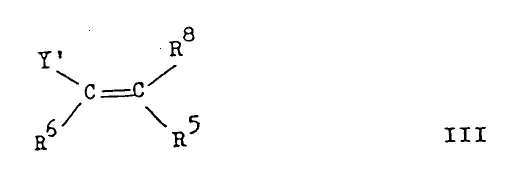 dans laquelle Y' et R⁵ ont la définition donnée ci-dessus, R⁶ représente un radical cyano ou un radical alkanoyle linéaire ou ramifié contenant de 2 à 5 atomes de carbone et R⁸ représente un radical alkoxy linéaire ou ramifié contenant de 1 à 4 atomes de carbone, de préférence un radical ethoxy, le radical hydroxy ou un atome de fluor, de chlore ou de brome.
(ii) Lorsque dans la formule générale IA, Z' représente un radical amino non substitué, la réaction d'un composé de formule générale Y'CH₂CN avec un composé de formule générale II en présence d'un composé de formule générale R⁷C(R^{o})₃ dans laquelle R⁷ représente un radical cycloalkyle contenant de 3 à 6 atomes de carbone et R^{o} représente un radical alkoxy, dans un solvant organique inerte, à une température comprise entre la température ambiante et la température de reflux, la préparation des autres composés de formule générale I se faisant par conversion, comme décrit précédemment, de un ou plusieurs des substituants Y, Z, R³, R⁴, ou des substituants correspondants, en substituants Y, Z, R³ et R⁴ tels que définis dans la revendication 1; et, lorsque Z représente le radical carboxy, en transformant éventuellement le composé de formule générale I en l'un de ses sels.

9. Un composé de formule générale I comme défini dans la revendication 1, ou un sel acceptable comme pesticide de ces composés, pour l'utilisation dans la fabrication d'un médicament pour le traitement des infections dues aux arthropodes ou aux helminthes.

## Revendications (Revendications pour l'(les) Etat(s) contractant(s) suivant(s): AT, ES)

1. Procédé de préparation d'un composé de formule générale : 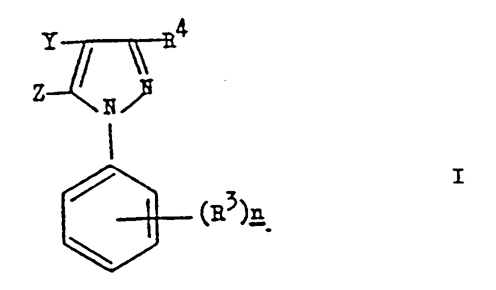 dans laquelle Y représente un atome d'halogène, le groupe cyano ou un groupe RSO₂, RSO ou RS dans lequel R représente un groupe alkyle à chaîne droite ou ramifiée contenant 1 à 6 atomes de carbone qui est non substitué ou substitué avec un ou plusieurs atomes d'halogènes, un groupe cycloalkyle contenant 3 à 5 atomes de carbone, un groupe alcényle à chaîne droite ou ramifiée contenant 2 à 6 atomes de carbone, le groupe thiocyanato, le groupe sulfamoyle qui est non substitué ou substitué avec un ou deux groupes alkyle à chaîne droite ou ramifiée qui peuvent être identiques ou différents et qui contiennent 2 à 6 atomes, le groupe carbamoyle qui peut être non substitué ou substitué avec un ou deux groupes alkyle à chaîne droite ou ramifiée qui peuvent être identiques ou différents et qui contiennent 1 à 6 atomes de carbone, un groupe alkoxycarbonyle à chaîne droite ou ramifiée contenant 2 à 7 atomes de carbone, un groupe alcanoyle à chaîne droite ou ramifiée contenant 2 à 7 atomes de carbone ou un groupe alkyle à chaîne droite ou ramifiée contenant 1 à 6 atomes de carbone qui est non substitué ou substitué avec un ou plusieurs atomes d'halogènes, Z représente l'atome d'hydrogène ou un groupe amino -NR¹R² dans lequel R¹ et R², qui peuvent être identiques ou différents, représentent chacun un atome d'hydrogène ou un groupe alkyle à chaîne droite ou ramifiée (contenant 1 à 6 atomes de carbone, et qui est non substitué ou substitué avec un groupe alkoxycarbonyle à chaîne droite ou ramifiée contenant 2 à 5 atomes de carbone), un groupe cycloalkyle contenant 3 à 6 atomes de carbone, un groupe formyle, un groupe alcanoyle à chaîne droite ou ramifiée (qui contient 2 à 7 atomes de carbone, ou bien ces groupes forment conjointement avec l'atome d'azote auquel ils sont fixés, un imide cyclique penta- ou hexagonal et sont eux-mêmes non substitués ou substitués avec un ou plusieurs atomes d'halogènes) ou un groupe cycloalkylcarbonyle (qui contient 4 à 7 atomes de carbone) ou un groupe alkoxycarbonyle à chaîne droite ou ramifiée (qui contient 2 à 7 atomes de carbone, ces groupes étant eux-mêmes non substitués ou substitués avec un ou plusieurs atomes d'halogènes), ou Z représente un groupe alkylsulfénylamino à chaîne droite ou ramifiée contenant 1 à 4 atomes de carbone, un groupe alkoxyméthylèneamino à chaîne droite ou ramifiée contenant 2 à 5 atomes de carbone qui est non substitué ou substitué sur le groupe méthylène avec un groupe alkyle à chaîne droite ou ramifiée contenant 1 à 4 atomes de carbone, ou représente un atome d'halogène, un groupe alkyle à chaîne droite ou ramifiée contenant 1 à 4 atomes de carbone, un groupe carboxy ou un groupe alkylthio, alkylsulfinyle ou alkylsulfonyle à chaîne droite ou ramifiée contenant 1 à 6 atomes de carbone qui est non substitué ou substitué avec un ou plusieurs atomes d'halogènes, ou représente un groupe trialkylsilylméthyle à chaîne droite ou ramifiée contenant 1 à 6 atomes de carbone dans chaque groupe alkyle, lesdits groupes alkyle pouvant être identiques ou différents, un groupe trialkylsilyle contenant 1 à 6 atomes de carbone dans chaque groupe alkyle, lesdits groupes alkyle pouvant être identiques ou différents, ou un groupe cyano ou nitro, R³ représente un atome d'halogène, un groupe alkyle ou alkoxy à chaîne droite ou ramifiée contenant 1 à 4 atomes de carbone qui est non substitué ou substitué avec un ou plusieurs atomes d'halogènes, un groupe alkylthio ou alkylsulfinyle à chaîne droite ou ramifiée contenant 1 à 4 atomes de carbone qui est substitué avec un ou plusieurs atomes d'halogènes, un groupe nitro ou cyano ou un groupe alkylsulfonyle à chaîne droite ou ramifiée contenant 1 à 4 atomes de carbone qui est non substitué ou substitué avec un ou plusieurs atomes d'halogènes, et R⁴ représente un groupe cycloalkyle contenant 3 à 6 atomes de carbone, et n représente un nombre entier de 1 à 5 inclus, et, lorsque Z représente un groupe carboxy, de ses sels formés avec des bases acceptables du pointe de vue pesticide, procédé qui comprend, lorsque le composé de formule générale I répond à la formule générale : 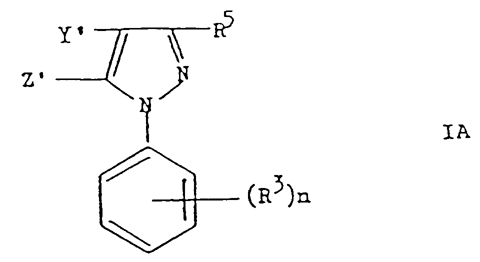 dans laquelle Y' représente le groupe cyano ou un groupe RSO₂, RSO ou RS, dans laquelle R répond à la définition précitée, un groupe alkoxycarbonyle à chaîne droite ou ramifiée contenant 2 à 7 atomes de carbone, ou un groupe alkyle à chaîne droite ou ramifiée contenant 1 à 6 atomes de carbone qui est non substitué ou substitué avec un ou plusieurs atomes d'halogènes, Z' représente le groupe amino non substitué ou un groupe alkyle à chaîne droite ou ramifiée contenant 1 à 4 atomes de carbone, et R⁵ représente un groupe cycloalkyle contenant 3 à 6 atomes de carbone :
(i) la réaction d'un composé de formule générale : 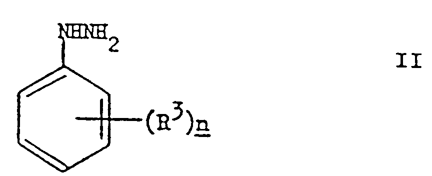 dans laquelle R³ et n répondent aux définitions précitées, ou d'un de ses sels d'addition d'acide, avec un composé de formule générale : 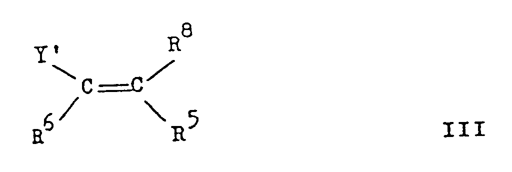 dans laquelle Y' et R⁵ répondent aux définitions précitées, R⁶ représente le groupe cyano ou un groupe alcanoyle à chaîne droite ou ramifiée contenant 2 à 5 atomes de carbone et R⁸ représente un groupe alkoxy à chaîne droite ou ramifiée contenant 1 à 4 atomes de carbone, le groupe hydroxy ou un atome de fluor, de chlore ou de brome, ou
(ii) lorsque Z' dans la formule générale IA représente un groupe amino non substitué, la réaction d'un composé de formule générale Y'CH₂CN avec un composé de formule générale II en présence d'un composé de formule générale R⁷C(R⁰)₃ dans laquelle R⁷ représente un groupe cycloalkyle contenant 3 à 6 atomes de carbone et R⁰ représente un groupe alkoxy dans un solvant organique inerte à une température comprise dans l'intervalle de la température ambiante à la température du reflux ; et la préparation d'autres composés de formule générale L par conversion, de la manière décrite précédemment, d'un ou plusieurs substituants Y, Z, R³ et R⁴, ou de substituants correspondants à ceux-ci, en substituants Y, Z, R³ et R⁴ répondant aux définitions précitées ; et, lorsque Z représente un groupe carboxy, la conversion facultative d'un composé de formule générale I en un de ses sels.

2. Procédé suivant la revendication 1, dans lequel, dans la formule générale L, (R³)ₙ représente un substituant 2,4,6-trichloro, 2,3,5,6-tétrachloro, 2-chloro-4-trifluorométhyle, 2,3,5,6-tétrafluoro-4-trifluorométhyle, 2,6-dichloro-4-trifluorométhylthio, 2-chloro-3,5,6-trifluoro-4-trifluorométhyle, 2,6-dichloro-3,5-difluoro-4-trifluorométhyle, 2,6-dichloro-4-nitro, 2,6-dichloro-4-trifluorométhylsulfinyle, 2,6-dichloro-4-méthanesulfonyle ou 2,6-dichloro-4-trifluorométhanesulfonyle.

3. Procédé suivant la revendication 1, dans lequel, dans la formule I, (R³)ₙ représente un substituant 2,6-dichloro-4-trifluorométhyle ou 2,6-dichloro-4-trifluorométhoxy.

4. Procédé de préparation d'une composition destinée à lutter contre des arthropodes, des nématodes de végétaux, ou des helminthes, qui comprend la formation d'au moins un composé de formule générale I répondant à définition suivant la revendication 1, ou d'un de ses sels acceptables du point de vue pesticide, avec un ou plusieurs diluants ou supports compatibles.

5. Procédé pour lutter contre des arthropodes, des nématodes de végétaux ou des helminthes parasites dans un milieu, qui comprend le traitement de ce milieu avec une quantité efficace d'un composé de formule générale I répondant à la définition suivant la revendication 1, ou d'un de ses sels, à l'exclusion d'un procédé pour le traitement de l'organisme de l'homme ou d'un animal effectué par un médecin ou un vétérinaire à titre thérapeutique.

6. Procédé suivant la revendication 5, dans lequel (R³)ₙ représente un substituant 2,4,6-trichloro, 2,3,5,6-tétrachloro, 2-chloro-4-trifluorométhyle, 2,3,5,6-tétrafluoro-4-trifluorométhyle, 2,6-dichloro-4-trifluorométhylthio, 2-chloro-3,5,6-trifluoro-4-trifluorométhyle, 2,6-dichloro-3,5-difluoro-4-trifluorométhyle, 2,6-dichloro-4-nitro, 2,6-dichloro-4-trifluorométhylsulfinyle, 2,6-dichloro-4-méthanesulfonyle ou 2,6-dichloro-4-trifluorométhanesulfonyle.

7. Procédé suivant la revendication 5, dans lequel (R³)ₙ représente un substituant 2,6-dichloro-4-trifluorométhyle ou 2,6-dichloro-4-trifluorométhoxy.
